# EUROPÄISCHE PATENTSCHRIFT

(11) **EP 1 328 628 B1**
(45) Veröffentlichungstag und Bekanntmachung des Hinweises auf die Patenterteilung: **31.12.2008**
(21) Anmeldenummer: 01988769.4
(22) Anmeldetag: 25.10.2001
(51) Int. Cl.: C12N 15/10, C07K 14/245

(54) **VERFAHREN ZUR EXPOSITION VON PEPTIDEN UND POLYPEPTIDEN AUF DER ZELLOBERFLÄCHE VON BAKTERIEN**
METHOD FOR EXPOSING PEPTIDES AND POLYPEPTIDES ON THE CELL SURFACE OF BACTERIA
PROCEDE POUR L'EXPOSITION DE PEPTIDES ET DE POLYPEPTIDES SUR LA SURFACE CELLULAIRE DE BACTERIES

(30) Priorität: 26.10.2000 DE 10053224
(43) Veröffentlichungstag der Anmeldung: 23.07.2003
(73) Patentinhaber: NascaCell Technologies AG, 81377 München (DE)
(72) Erfinder: KOLMAR, Harald, 64367 Mühltal (DE); CHRISTMANN, Andreas, 37081 Göttingen (DE); WENTZEL, Alexander, 7020 Trondheim (NO)
(74) Vertreter: Bohmann, Armin K.
(86) Internationale Anmeldenummer: PCT/DE2001/004009
(87) Internationale Veröffentlichungsnummer: WO 2002/034906

(56) Entgegenhaltungen:
- EP-A- 0 872 555
- CHRISTMANN ANDREAS ET AL: "The cystine knot of a squash-type protease inhibitor as a structural scaffold for Escherichia coli cell surface display of conformationally constrained peptides" PROTEIN ENGINEERING, OXFORD UNIVERSITY PRESS, SURREY, GB, Bd. 12, Nr. 9, 1999, Seiten 797-806, XP002175661 ISSN: 0269-2139 in der Anmeldung erwähnt
- SCHEMBRI & KLEMM: "Hetterobinary adhesins based on the Escherichia coli FimH fimbrial protein" APPLIED AND ENVIRONMENTAL MICROBIOLOGY, WASHINGTON,DC, US, Bd. 64, Nr. 5, Mai 1998 (1998-05), Seiten 1628-1633, XP002109166 ISSN: 0099-2240
- DATABASE [Online] 11. Oktober 2001 (2001-10-11) KOLMAR, H.: "pASKInt100 - Expression vector for surface display of Intimin fusion proteins" retrieved from WWW.GWDG.DE/~HKOLMAR/PASKINT100.HTM XP002211330
- CHRISTMANN A ET AL: "Epitope mapping and affinity purification of monospecific antibodies by Escherichia coli cell surface display of gene-derived random peptide libraries" JOURNAL OF IMMUNOLOGICAL METHODS, ELSEVIER SCIENCE PUBLISHERS B.V.,AMSTERDAM, NL, Bd. 257, Nr. 1-2, 1. November 2001 (2001-11-01), Seiten 163-173, XP004311948 ISSN: 0022-1759
- WENTZEL ALEXANDER ET AL: "Display of passenger proteins on the surface of Escherichia coli K-12 by the enterohemorrhagic E. coli intimin EaeA." JOURNAL OF BACTERIOLOGY, Bd. 183, Nr. 24, Dezember 2001 (2001-12), Seiten 7273-7284, XP002211329 ISSN: 0021-9193
- KELLY G. ET AL: "STRUCTURE OF THE CELL-ADHESION FRAGMENT OF INTIMIN FROM ENTEROPATHOGENIC ESCHERICHIA COLI" NATURE STRUCTURAL BIOLOGY, Bd. 6, Nr. 4, April 1999 (1999-04), Seiten 313-318, NEW YORK
- HUGO N. ET AL.: "Functional aspects of co-variant surface charges in an antibody fragment" PROTEIN SCIENCE, Bd. 11, Nr. 11, November 2002 (2002-11), Seiten 2697-2705,
- RIPPMANN J. ET AL.: "Procaryotic expression of single-chain variable-fragment (scFv) antibodies: Secretion in L-form cells of Proteus mirabilis leads to active product and overcomes the limitations of periplasmic expression in Escherichia coli" APPLIED AND ENVIRONMENTAL MICROBIOLOGY, Bd. 64, Nr. 12, 1. Dezember 1998 (1998-12-01), Seiten 4862-4869,
- DERSCH P. ET AL.: "An immunoglobulin superfamily-like domain unique to the Yersinia pseudotuberculosis invasin protein is required for stimulation of bacterial uptake via integrin receptors" INFECTION AND IMMUNITY, Bd. 68, Nr. 5, Mai 2000 (2000-05), Seiten 2930-2938,
- SKERRA A.: "Use of the tetracycline promoter for the tightly regulated production of a murine antibody fragment in Escherichia coli" GENE, Bd. 151, Nr. 1, 30. Dezember 1994 (1994-12-30), Seiten 131-135,
- BATCHELOR M. ET AL.: "Structural basis for recognition of the translocated intimin receptor (Tir) by intimin from enteropathogenic Escherichia coli" EMBO JOURNAL, Bd. 19, Nr. 11, 1. Juni 2000 (2000-06-01), Seiten 2452-2464,
- ADAMS T. ET AL.: "Intimin-mediated export of passenger proteins requires maintenance of a translocation-competent conformation" JOURNAL OF BACTERIOLOGY, Bd. 187, Nr. 2, 1. Januar 2005 (2005-01-01), Seiten 522-533,

## Beschreibung

Die vorliegende Erfindung betrifft ein Verfahren zur Präsentation von Peptiden, einschließlich Polypeptiden und Proteinen, je nach Länge der präsentierten Sequenz, auf der Oberfläche gramnegativer Bakterienzellen, insbesondere *Escherichia coli* Zellen, ein zugehöriges Verfahren zur Herstellung einer varianten Population von oberflächenexponierten Peptiden oder Polypeptiden und zur Identifizierung von Bakterien, die Peptide bzw. Polypeptide mit einer jeweils gewünschten Eigenschaft tragen, sowie bei dem Verfahren verwendbare Vektoren und Wirtsbakterien.

Generell erlaubt das Verfahren die Expression von rekombinanten Proteinen, die Rezeptoren oder Liganden sein können, auf der Bakterienoberfläche, sowie die Selektion aufgrund der Bindungsaffinität zu einem Bindungspartner. Das Verfahren erlaubt die Expression von Peptid- und Polypeptidbibliotheken auf der Oberfläche von Bakterienzellen, mit deren Hilfe beispielsweise Peptidmoleküle mit hoher Affinität zu einem Liganden identifiziert werden können. Das Verfahren gestattet die Präsentation einer großen Anzahl von Passagierproteinen bzw. -peptiden auf der Oberfläche einer Bakterienzelle, ohne daß die zelluläre Überlebensfähigkeit dadurch beeinflußt wird. Darüberhinaus ermöglicht dieses Verfahren, die Anzahl der pro Zelle oberflächenpräsentierten Moleküle nach Wunsch einzustellen.

Insbesondere betrifft die vorliegende Erfindung auch die Isolierung von monospezifischen Antikörpern aus polyklonalen Antikörpergemischen durch Bindung an Zelloberflächenverankerte Peptidepitope.

### Hintergrund

Gegenwärtig wird die Expression von Proteinen und Proteindomänen auf der Oberfläche von selbstreplizierenden Trägem wie Bakteriophagen, Bakterien, Hefen usw. intensiv untersucht, vor allem mit der Zielsetzung, eine Kopplung der funktionalen Ausprägung einer Eigenschaft des auf dem Träger präsentierten Proteins (Phänotyp) mit der zugrundeliegenden genetischen Kodierung (Genotyp) zu erreichen. Beispiele für erfolgreiche Phänotyp/Genotyp Kopplung finden sich u.a. bei der Verwendung von Hefen als Träger Oberflächen-präsentierter Proteine. So wurde z.B. ein molekulares Repertoire an varianten Hefezellen erzeugt, welche jeweils verschiedene Immunglobulinfragmente auf ihrer Zelloberfläche präsentieren. Aus dieser Bibliothek von Antikörpervarianten konnten solche isoliert werden, die eine erhöhte Affinität zu einem vorgegebenen Ligandenmolekül aufwiesen (Boder und Wittrup (1997), Nat. Biotechnol. 15: 1553)

Eine breite Anwendung finden Bakterien bei der Zelloberflächenpräsentation. Es kommen sowohl Gram-negative als auch Gram-positive Arten zur Anwendung. So konnten auf *Staphylococcus xylosus* und *Staphylococcus carnosus* Proteine durch *Staphylococcus aureus* Protein A präsentiert werden. Durch die Fusion an *Staphylococcus aureus* Fibronectin Bindeprotein B (FnBPB) konnten Enzyme auf der Oberfläche von *S. carnosus* verankert werden (Strauss und Götz (1996), Mol. Microbiol. 21: 491-500). Gram-positive Bakterien sind jedoch für die Präsentation großer Peptidbibliotheken wenig geeignet, da es schwierig ist, die entsprechenden Genvarianten durch Transformation in die Zellen einzubringen und eine hinreichend große Anzahl unabhängiger und bezüglich der kodierenden Nukleotidsequenz der Oberflächen-präsentierten Proteinvariante unterschiedlicher Klone zu erzeugen.

Gram-negative Bakterien hingegen sind zur Generierung molekularer Repertoires und der damit einhergehenden Präsentation veränderter Proteine aufgrund der hohen Transformationsausbeute (>10⁹ pro Mikrogramm Plasmid DNA bei *E. coli* sehr gut geeignet und daher bevorzugte Wirtsorganismen.

Für die Präsentation rekombinanter Proteine auf der Zelloberfläche Gram-negativer Bakterien wurden verschiedene Systeme beschrieben (Georgiou et al. (1997), Nature Biotechnol. 15: 29-34). Eine Oberflächenpräsentation wird im allgemeinen dadurch erreicht, daß Gensegmente eines bakteriellen Oberflächenproteins mit dem Gen des zu präsentierenden Proteins fusioniert werden. Dabei kommen üblicherweise Proteine als Träger zum Einsatz, die ihrerseits sekretiert werden und/oder in der äußeren Membran Gram-negativer Bakterien lokalisiert sind und somit die notwendigen Signale zur Translokation durch die Cytoplasmamembran, die Passage in das bakterielle Periplasma und die Integration in die äußere Membran oder die Verankerung auf der Oberfläche der äußeren Membran beinhalten. Als Trägerproteine kamen bislang zumeist Proteine zum Einsatz, die ihrerseits integrale Bestandteile der äußeren Membran von *E. coli* sind. Dazu gehören unter anderem, PhoE (Agterberg et al. (1987), Gene 59: 145-150) oder OmpA (Francisco et al. (1992), Proc. Natl. Acad. Sci. USA 89: 2713-2717), deren Verwendung jedoch Nachteile aufweisen. So können Proteinsequenzen nur in oberflächenexponierten Schleifen dieser Proteine inseriert werden, was zum einen zu konformationell fixierten Amino- und Carboxytermini Anlass gibt und zum anderen die Länge der zu inserierenden Peptidsequenz drastisch einschränkt. Die Verwendung des Peptidoglycan-assoziierten Lipoproteins (PAL) als Trägerprotein führt zwar zum Transport zur äußeren Membran, eine Präsentation aktiver und korrekt gefalteter Proteinsequenzen auf der Oberfläche von E. *coli* ist jedoch nicht möglich (Fuchs et al. (1991), Bio. Technology 9, 1369-1372). Eine Oberflächenexpression größerer Proteine gelang (a) durch Verwendung einer Fusion aus einem Fragment des *Escherichia coli* Lpp und des OmpA Proteins als Trägerproteinanteil, an deren Carboxy-Ende die Passagierproteinsequenz angehängt wird (Francisco et al. (1992), Proc. Natl. Acad. Sci. 89: 2713-2717) (b) durch Verwendung der IgA-Protease β-Domäne (IgAß) und anderer bakterieller Autotransporter (Maurer et al. (1997) J. Bacteriol. 179: 794-804), sowie (c) durch die Verwendung des Eis-Nukleationsproteins von *Pseudomonas syringae* (InaZ) (Jung et al. (1998), Nature Biotechnol. 16: 576-580) als Trägerproteinanteil.

Aus den obigen Beispielen wird deutlich, daß eine Präsentation von Proteinen auf der bakteriellen Zelloberfläche dadurch hervorgerufen werden kann, daß eine Passagierdomäne durch Fusion der entsprechenden kodierenden DNA-Sequenz mit der kodierenden Sequenz eines ausgewählten Proteins der äußeren Membran oder Membranproteinfragments der äußeren Membran an ein Trägerprotein fusioniert wird. Das Membranprotein oder Membranproteinfragment trägt dabei die zur Membranlokalisation und -verankerung notwendigen Motive. Das Trägerprotein der äußeren Membran sollte dabei (a) ein Sekretionssignal besitzen, das eine Passage durch die Cytoplasmamembran sicherstellt, (b) ein Lokalisationssignal für die Einbettung in die äußere Membran aufweisen, (c) in möglichst hoher Kopienzahl auf der Zelloberfläche auftreten und (d) die strukturelle und funktionelle Integrität und vor allem die Vitalität der Wirtszelle nicht negativ beeinflussen.

Bei den nach Stand der Technik beschriebenen Verfahren zur Herstellung heterologer Passagierproteine mit Hilfe von Proteinen der äußeren Membran wurden jedoch erhebliche Nachteile, vor allem bezüglich der beiden Anforderungen (c) und (d) festgestellt, da eine hohe Expressionsrate und eine hohe Nettoakkumulation in der äußeren Membran stets mit einer hohen Mortalität der präsentierenden Bakterienzellen einhergeht. So ist eine starke Überexpression von Fusionsproteinen mit Lpp-OmpA als Membrananker lethal (Daugherty et al. (1999), Protein Eng. 12: 613-21). Eine hohe Zellmortalität wurde ebenfalls für die Verwendung des Autotransporters der IgA-Protease (IgAß) beschrieben (Wentzel et al. (1999), J. Biol. Chem. 274: 21037-21043). Jung *et al.* führten das Eis-Nukleationsprotein aus *Pseudomonas syringae,* das ein Glycosyl-Phosphatidylinositol verankertes Protein der äußeren Membran ist, als Trägerprotein für die Zelloberflächenpräsentation von Passagierproteinen in *E. coli* ein (Jung et al. (1998), Nature Biotechnol. 16: 576-580). Dieses Trägerprotein erlaubt zwar die stabile Präsentation von Passagieren auf der Oberfläche der äußeren Membran, die Fusionsproteine aggregieren jedoch in Clustern auf der bakteriellen Oberfläche, eine Eigenschaft, die für Zwecke der Selektion von Peptiden und Polypeptiden mit hoher Affinität zu einem gegebenen Bindungspartner unerwünscht ist.

Neben den integralen Proteinen der äußeren Membran wurden noch andere Oberflächenstrukturen, die auf der Zelloberfläche vorhanden sind, wie Flagellen, Pili, Fimbrien u.s.w. als Träger für die Präsentation von Passagierdomänen herangezogen. Durch die Verwendung von Flagellin, einer strukturellen Untereinheit des Flagellums als Träger konnten diverse Peptide des Hepatitis B Virus stabil exprimiert und auf der Bakterienoberfläche exponiert werden (Newton et al. (1989), Science 244: 70-72). Ebenso wie bei der Verwendung von Fimbrin als strukturellem Trägerprotein bleibt die Präsentation von Passagierdomänen jedoch auf kleine Peptide beschränkt (Hedegaard et al (1989), Gene 85: 115-124).

### Technisches Problem und dessen Lösung durch die vorliegende Erfindung

Der vorliegenden Erfindung lag also das technische Problem zugrunde, Trägerproteine bereitzustellen, die insbesondere bei Verwendung von *Escherichia coli* als Wirtsorganismus nicht zu den oben genannten Nachteilen führen.

### Ein optimales Präsentationsverfahren muß folgende Aufgaben erfüllen:

1. Das zu präsentierende Peptid/Protein soll vorzugsweise in möglichst hoher Kopienzahl verankert auf der Oberfläche einer Bakterienzelle vorliegen.
2. Die Überlebensfähigkeit soll durch die Peptid/Protein-Präsentation nicht beeinträchtigt werden.
3. Die Anzahl der pro Zelle oberflächenpräsentierten Peptid/Proteinmoleküle sollte in weiten Grenzen regulierbar sein.

Ein Verfahren zur bakteriellen Oberflächenpräsentation, welches diese Anforderungen in allen Punkten erfüllt, ist bisher nicht beschrieben worden.

### Zusammenfassung der Erfindung

Zur Lösung der vorstehenden Aufgabe ist bei einem Verfahren zur Präsentation von Peptiden und/oder Polypeptiden auf der Oberfläche von Wirtsbakterien vorgesehen, dass man (a) ein gramnegatives Wirtsbakterium bereitstellt, das transformiert ist mit einem Vektor, auf dem in operativer Verknüpfung mit einem exogen induzierbaren Promotor eine fusionierte Nukleinsäuresequenz lokalisiert ist, welche: (i) einen Sequenzabschnitt, der ein carboylterminalen Bereich um wenigstens die D3-Domäne verkürztes Intimin kodiert, als Verankerungsdomäne und (ii) einen für das zu präsentierende Passagierpeptid und/oder Passagierpolypeptid kodierenden Nukleinsäuresequenzabschnitt aufweist, und (b) das Wirtsbakterium unter Bedingungen kultiviert, bei denen eine Expression der fusionierten Nukleinsäuresequenz und eine Präsentation des von dem Nukleinsäuresequenzabschnitt (ii) kodierten Peptids oder Polypeptids an der Oberfläche des Wirtsbakteriums erfolgt, wobei der Nukleinsäuresequenzabschnitt (ii) gegenüber dem für die Intimin-Membran-Verankerungsdomäne kodierenden Nukleinsäuresequenzabschnitt heterolog ist.

Das verkürzte intimin kann im carboxylterminalen Bereich von 280 Aminosäuren um wenigstens eine weitere der Domänen D0, D1 und/oder D2 verkürzt sein (neben der Verkürzung um die D3-Domäne).

Das erfindungsgemäß Verfahren ermöglicht die Präsentation von Peptiden oder Polypeptiden auf der Oberfläche von gramnegativen Wirtsbakterien mit Hilfe bestimmter Intimin-basierter Verankerungsmodule. Es wurde gefunden, dass sich carboxylterminal verkürzte Intimine besonders gut als Verankerungseinheiten für Passagierdomänen in der äußeren E. coli Zellmembran eignen.

Die Erfindung basiert auf der Bereitstellung eines Genkonstruktes, bei dem ein spezielles Trägerprotein (ein Fragment des sog. Intimins s.u.) als Präsentationsanker verwendet wird. Zur Präsentation eines vorgebenen Peptids/Proteins wird das kodierende Gen an die kodierende Sequenz des Intimin-Genfragments im durchgehenden Leseraster fusioniert und dadurch das resultierende Fusionsprotein in der äußeren Membran verankert präsentiert. Überraschenderweise zeigt sich bei Verwendung eines Intiminfragments als Membrananker, dass eine sehr große Anzahl von Molekülen in der Bakterienmembran bereitgestellt werden kann, ohne dass die Überlebensfähigkeit der Wirtsbakterien dadurch negativ beeinflusst wird. (b) Es ist uns gelungen, durch kombinierte Regulation der Expression des Intimingens auf Transkriptions- und Translationsebene die Anzahl der Moleküle pro Zelle nach Wunsch einzustellen. Bisher beschriebene Verfahren zur Regulation der Genexpression zum Zwecke der Einstellung oberflächenpräsentierter Moleküle leisten dies nicht.

### Intimin als Präsentationsanker

In Weiterbildung der Erfindung ist vorgesehen, dass die verwendete Intimin-Verankerungsdomäne in der äußeren Bakterienmembran aus der Gattung der Enterobacteriaceae abgeleitet wurde und in einem Wirtsbakterium einer Gattung der Enterobacteriaceae verwendet wird. Weiter vorzugsweise ist die Verankerungsdomäne ein Fragment des Intimins aus enterohämorrhagischen E. coli oder einer Variante von diesen. Besonders bevorzugt ist derzeit eine carboxylterminal verkürzte Variante des EaeA-Intimins aus enterohämorrhagischen Escherichia coli 0157:H7, das die Aminosäuren 1 bis 659 oder alternativ die Aminosäuren 1 bis 753 umfasst.

Viele Stämme pathogener Bakterien besitzen Oberflächenstrukturen, wie Pili, Glycoproteine und andere Proteine, ebenso wie homopolymere und heteropolymere Kohlenhydrat-Glycokalices, mit denen sich Bakterien an eukaryontische Zelloberflächen anheften. Proteine der bakteriellen Zelloberfläche, die als Intimine bezeichnet werden, spielen sich wichtige Rolle bei der festen Anheftung von enteropathogenen (EPEC) oder enterohämorrhagischen E. coli Bakterien (EHEC) an die Oberfläche eukaryontischer Wirtszellen. Die Intimin-vermittelte Anheftung erlaubt den Bakterien eine Vermehrung auf der Oberfläche der besiedelten Zellen. Intimin ist ein Vertreter einer Familie bakterieller Adhäsionsmoleküle, die im Bereich der aminoterminalen Region Sequenzhomologien untereinander aufweisen (McGraw et al. (199), Mol, Biol. Evol. 16:12-22).

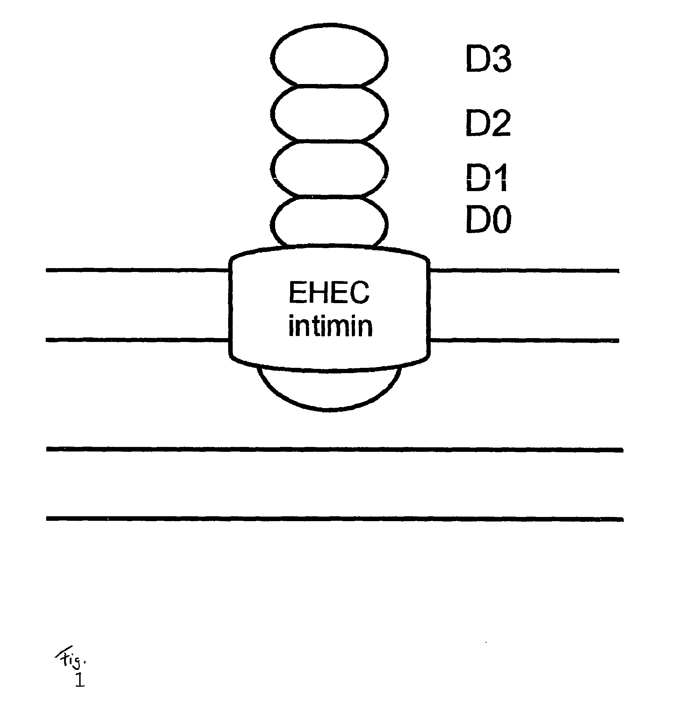

Die Zellbindeaktivität von Intimin, des Genprodukts des Eae Gens, welche 939 Aminosäurereste umfasst, ist in den C-terminalen 280 Aminosäureresten lokalisiert. Intimin ist mit seiner aminoterminalen Domäne in der äußeren Membran verankert, welche die carboxyterminalen 280 Aminosäuren exponiert. Diese 280 Reste kodieren drei Domänen, zwei Immunglobulin-ähnliche Domänen und eine Lektin-ähnliche Domäne (Kelly et al. (1999), Nat. Struct. Biol. 6:313-318), welche für die Bindung an Eukaryontenzellen verantwortlich sind. Die Struktur der Domänen D1, D2, D3 ist bekannt, nicht jedoch die Struktur der aminoterminalen Domäne (Kelly et al. (1999), Nat. Struct. Biol. 6:313-318). Der schematische Aufbau des Intimins und seine Verankerung in der äußeren Membran ist in Abb. 1 dargestellt. Entsprechende Domänen anderer Intimine lassen sich aus Sequenzvergleichen ableiten.

Intimine zeigen mit Ausnahme der aminoterminalen 200 Aminosäurereste Sequenzhomologien zu Invasinen aus Yersinien und anderen Bakterien, welche den Eintritt des Bakteriums in kultivierte Säugerzellen über die Bindung an Integrine ermöglichen (Leong et al. (1990), EMBO J. 9:1979-1989). In der EP 872 555 wird eine bakterielle peptide Library, die Invasinproteine als Trägermoleküle nutzen, beschrieben. Integrine und Invasine sind zwar sequenzähnlich, werden aber bezüglich ihrer Funktion verschiedenen Proteinfamilien zugeordnet (Batchelor et al. (2000), EMBO J. 19:2452-2464).

Durch die Verwendung eines Fragments des *Escherichia coli* Intimins als Transporterdomäne für die bakterielle Oberflächenlokalisation von Passagierproteinen und Passagierpeptiden konnte **überraschenderweise eine gegenüber dem Stand der Technik deutlich verbesserte Oberflächenpräsentation** von Peptiden oder Polypeptiden, insbesondere auch von kurzen synthetischen Peptiden mit einer Länge von vorzugsweise 6 bis 20 Aminosäuren, von disulfidverbrückten Peptiden und Polypeptiden, insbesondere Oligopeptiden auf der strukturellen Grundlage des Cystinknoten Faltungsmotivs (Pallaghy et al. (1994), Protein Sci. 3: 1833-1839) bzw. von bakteriellen (z.B: β-Laktamase-Inhibitorprotein) und eukaryontischen Polypeptiden (z.B. Interleukin 4) erreicht werden.

### Identifizierung des Eae Intimins als Trägerprotein für die Oberflächenexposition von Passagierproteinen

Das bevorzugt verwendete Escherichia coli Intimin ist in der äußeren Membran von *Escherichia coli* lokalisiert und exponiert von Natur aus mindestens eine Proteindomäne auf der Außenseite der äußeren Membran. Das *Escherichia coli* Intimin ist in der äußeren Membran verankert und trägt an seinem carboxyterminalen Ende vier Domänen, die für die Interaktion mit einem Rezeptorprotein auf der Oberfläche von Epithelzellen notwendig sind.

Ausgehend von der Arbeitshypothese, daß eine Substitution mindestens einer der Zelloberflächen-exponierten Intimindomänen durch eine heterologe Passagierdomäne zu einer Oberflächenexposition derselben führt, wurde eine Genfusion aus einem für ein Passagierprotein und einem für ein Intiminfragment kodierenden Nukleinsäuresequenzabschnitt hergestellt. Die Genfusion aus dem Intiminfragment und der Passagierdomäne, die Vektorkonstruktion und die Expression in Wirktsbakterien im Vergleich zu einem anderen Oberflächen-Präsentationsverfahren sind in Beispiel 1 dargestellt.

Generell wird für die Genfusion das gewählte Intimingen bzw. Genfragment durch Polymerase-Kettenreaktion amplifiziert und in einen für die Expression in dem vorgesehenen Wirtsbakterium geeigneten Vektor kloniert. In denselben Vektor wird stromabwärts von dem gewählten Intiminfragment in dasselbe Leseraster ein Gen für ein zu präsentierendes Passagierpeptid eingebracht. Der Vektor enthält außerdem einen exogen induzierbaren Promotor in operativer Verknüpfung mit dem Intimingen und dem damit fusionierten Passagierpeptidgen. Weitere funktionelle Sequenzen, z.B. Markergene, können ebenfalls vorhanden sein.

In Weiterbildung der Erfindung wird das Verfahren so ausgestaltet, dass die Expression des Fusionsgens aus dem für das verkürzte Intimin und dem für das Passagierprotein kodierenden Nukleinsäuresequenzabschnitt regulierbar ist, wobei (i) ein für ein Glutamin codierendes Codon des für das verkürzte Intimin kodierenden Nukleinsäuresequenzabschnitts durch ein amber Stopcodon (TAG) ersetzt ist und (ii) ein Escherichia coli Wirtsstamm verwendet wird, in dem eine Translation der mRNA des Fusionsgens durch Bereitstellung einer regulierbaren Menge an Suppressor-tRNA erfolgt, die ein Überlesen des Stopcodons bei der Translation ermöglicht. Dieses Verfahren ermöglicht eine wirksame Regulation der Expression des präsentierten Abschnitts innerhalb der Wirtszelle und ist bei praktisch allen bekannten Präsentationsverfahren nützlich.

Bei dem weitergebildeten Verfahren wird ein CAG-Codon innerhalb des Intiminfragments gegen ein TAG-Stopcodon ausgetauscht. Dies geschieht beispielsweise durch Klonierung eines PCR-Fragments, wobei das CAG-Codon Nummer #35 im Intimin gegen ein TAG-Stopcodon ausgetauscht wird. Bei Verwendung eines amber Suppressorstammes wird dieses Stopcodon überlesen und an dieser Stelle ein Glutaminrest eingebaut. Da die Effizienz der amber Suppression niedrig ist, werden dadurch weniger Moleküle synthetisert, als bei Abwesenheit des Stopcodons. Dadurch wird erreicht, dass die Zahl der oberflächenpräsentierten Moleküle in einem für die Zelle tolerablen Umfang bleibt (siehe hierzu Christmann et al. (1999), Protein Eng. 12: 797-806). Als Vektor kann jeder insbesondere zur Expression in E.coli verwendbare Expressionsvektor verwendet werden, als Ausgangsvektor geeignet ist u.a. der Vektor pASK75 (siehe unten).

### Regulation der Genexpression

Üblicherweise erfolgt die Regulation der Expression eines Genes dadurch, dass die kodierende Sequenz eines Gens unter die Kontrolle eines Promotors gebracht wird, bei dem die Anzahl der pro Zeiteinheit generierten Transkripte durch die Konzentration eines exogen zugesetzten Induktormoleküls reguliert werden kann. Hierfür kommen z.B. der *lac*-Promotor, der *ara*-Promotor oder der *tetA*-Promotor in Frage (Lutz &Bujard (1997, Nucleic Acids. Res. 25:1203). Eine Regulation der Genexpression mit dem Ziel der Einstellung einer erwünschten Anzahl an Oberflächen-präsentierten Molekülen pro Bakterienzelle durch kontrollierte Induktion der Transkription der Gene von Oberflächen-exponierten Fusionsproteinen ist bisher nicht befriedigend gelungen. Die Variation der Konzentration eines zugesetzten Induktors führte bisher nicht zu einer von der Konzentration des Induktors abhängigen Akkumulation von Fusionsproteinen auf der Oberfläche der Bakterienzelle (Daugherty et al. (1999), Protein Eng. 12: 613-621). Eine geringfügige Verbesserung wurde erzielt durch die Einstellung der Nettoakkumulation über die Induktionszeit. Das bedeutet, dass einer Bakterienkultur der Induktor der Transkription zugesetzt wird, worauf die Zellen verschieden lange mit Induktor inkubiert werden. Je länger die Induktionszeit ist, umso höher ist die Anzahl der oberflächenpräsentierten Zellen pro Zelle. Dieses Verfahren ist für hochparallele biotechnologische Anwendungen nicht geeignet, da es Probennahmen zu verschiedenen Wachstumszeiten erfordert, außerdem befinden sich die Zellen je nach Wachstumszeit und erreichter optischer Dichte in unterschiedlichen physiologischen Zuständen.

**Das von uns entwickelte Verfahren hebt diese Nachteile auf.** Die Kontrolle der Genexpression erfolgt auf zwei Ebenen, nämlich auf Ebene der Transkription und auf der Ebene der Translation.

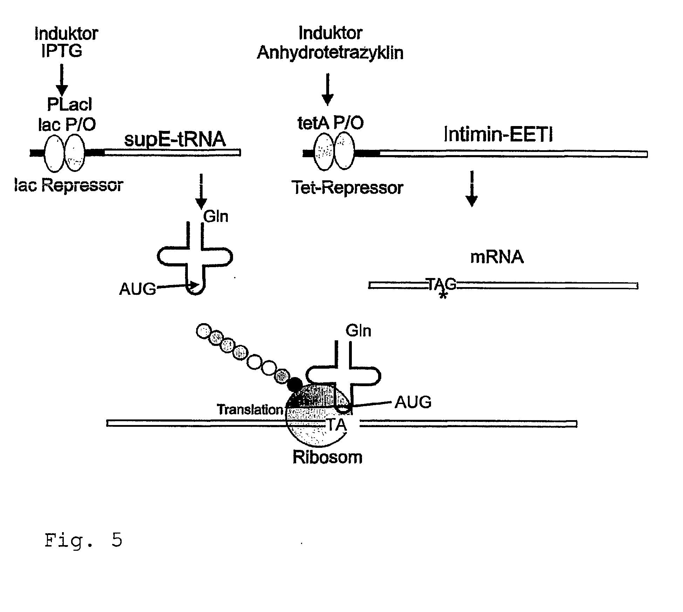

In einer erfindungsgemäßen Vorgehensweise werden dazu Fusionsproteine hergestellt, bei denen ein für Glutamin kodierendes Codon (CAG) des für das Intimin oder Intiminfragment kodierenden Nukleinsäuresequenzabschnitts durch ein *amber* Stopcodon (TAG) ersetzt ist. Dieses Codon ist bevorzugt das erste Glutamincodon der Aminosäuresequenz des Intimins oder Intiminfragments. Ebenso kann ein anderes Codon innerhalb der ersten 100 Aminosäuren des Intimins oder Intiminfragments gegen TAG ausgetauscht sein. Es wird nun *in trans* in einem *E. coli* Stamm eine modifizierte Glutaminyl-tRNA angeboten, die den genetischen Marker *supE* trägt. Diese supE tRNA ist in der Lage, bei der Translation mit einem TAG-Codon zu paaren und eine Suppression des Translationsstopps herbeizuführen. Erfindungsgemäß ist ein *E. coli* Wirtsbakterium, welches einen das *supE* Gen kodierenden Nukleinsäuresequenzabschnitt in operativer Verknüpfung mit einem regulierbaren Promotor enthält. Bevorzugt ist hierbei der PL*lac-Promotor,* bei dem die Expressionsstärke und damit die Syntheserate von *supE* Transfer-RNA durch die Menge an dem Wachstumsmedium der Wirtsbakterien zugesetzten Induktor IPTG reguliert werden kann. In einem typischen Beispiel ist der Nukleinsäuresequenzabschnitt, welcher für ein *supE* tRNA Gen in operativer Verknüpfung mit einem regulierbaren Promotor kodiert in einem Vektor lokalisiert, welcher zum Expressionsvektor kompatibel ist. Die Transkription des Gens für das Intimin-Passagierdomäne-Fusionsprotein wird durch Zugabe eines Induktors angeschaltet (hier Anhydrotetrazyklin). Durch Variation der Menge an IPTG-Induktor werden unterschiedliche und frei einstellbare Mengen an Suppressor tRNA zur Synthese der Intimin-Fusionsproteine in der Zelle zur Verfügung gestellt. Die Menge an bereitgestellter supE tRNA determiniert letztlich die Anzahl der durchschnittlich auf der Oberfläche einer Bakterienzelle präsentierten Passagierdomänen. Das neu entwickelte Expressionsverfahren ist in Abb. 5 schematisch dargestellt.

### Ausführungsformen

Beim erfindungsgemäßen Verfahren wird ein Wirtsbakterium bereitgestellt, das mit einem bzw. mehreren kompatiblen Vektoren transformiert ist. Ein solcher Vektor enthält in operativer Verknüpfung mit einem Promotor und gegebenenfalls weiteren für die Expression erforderlichen Sequenzen eine fusionierte Nukleinsäuresequenz. Diese fusionierte Nukleinsäuresequenz umfaßt (a) einen ein Intiminfragment kodierenden Nukleinsäuresequenzabschnitt, welcher eine Präsentation des von Abschnitt (b) kodierten Peptids oder Polypeptids auf der Außenseite der äußeren Membran des Wirtsbakteriums ermöglicht und (b) einen das zu präsentierende Protein und oder Peptid kodierenden Nukeinsäuresequenzabschnitt.

In einer bevorzugten Ausführungsform betrifft die vorliegende Erfindung daher ein Intimin, ein Fragment des Intimins oder ein zu Intimin homologes Trägerprotein, das eine Transporterfunktion ausübt und eine Oberflächenexposition von rekombinanten Proteinen in Wirtsbakterien **in hoher Kopienzahl** ermöglicht. Hierbei handelt es sich um das aminoterminale Fragment des Eae Intimins aus enterohämorrhagischen *Escherichia coli* Serotyp O157:H7 (Louie *et al.* (1993), 61: 4085-4092), welches von Aminosäure 1 bis 659 reicht. Neben dieser spezifischen Sequenz ist auch die Verwendung von Varianten erfindungsgemäß, die beispielsweise durch Veränderung oder Deletion der Aminosäuresequenz in den nicht für die Translokation durch die Cytoplasmamembran und Lokalisation in der äußeren Membran notwendigen Sequenzabschnitten erzeugt werden können.

In einem weiteren typischen Beispiel handelt es sich bei dem verwendeten Transporterprotein um das gamma Intimin aus *E. coli* (Genbank Accession Nr. (AF081182), das Intimin aus *E. coli* O111:H- (Genbank Accession Nr. AAC69247) oder Intimin aus anderen *Escherichia coli* Serotypen oder das Intimin aus *Citrobacter freundii* (Genbank Accession Nr. AAA23097). Die DNA Sequenzen und die davon abgeleiteten Aminosäuresequenzen der zuvor genannten Intimine sind an den unten angeführten Stellen als NCBI-Zitate (National Center for Biotechnology Information, U.S.A.) zu finden.

Weitere Intimindomänen können aus in Datenbanken vorliegenden Proteinsequenzen, aus Proteinsequenzen, die auf in Datenbanken verfügbaren DNA-Sequenzen basieren, oder aus durch Sequenzanalyse direkt oder indirekt über die DNA-Sequenz bestimmten Proteinsequenzen abgeleitet werden. Die entsprechenden kodierenden Regionen (Gene) können zur Herstellung von Vektoren oder Fusionsproteingenen, die eine effektive Oberflächenexpression von Passagierproteinen in Gram-negativen Bakterien, insbesondere *Escherichia coli* ermöglichen, verwendet werden.

Oberflächenpräsentation bzw. -exposition bedeutet erfindungsgemäß, dass die Fusionsproteine bzw. Passagierdomänen auf der dem Medium zugewandten Seite der äußeren Bakterienmembran lokalisiert sind. Oberflächenexponierte Passagierproteine sind in intakten Gram-negativen Bakterien frei zugänglich für Bindungspartner.

In einer bevorzugten Ausführungsform ermöglicht die vorliegende Erfindung also die Oberflächenpräsentation von Peptiden oder in einer weiteren Ausführungsform die Oberflächenpräsentation von Peptidbibliotheken in Gram-negativen Bakterien, insbesondere in *E. coli* und deren Verwendung zur Bestimmung der Affinität zu einem Antikörper oder einem anderen Rezeptor.

In einer weiteren bevorzugten Ausführungsform ermöglicht die vorliegende Erfindung die Kartierung von Epitopen und die Isolierung monospezifischer Antikörper aus einem Antikörpergemisch. Epitopkartierung bedeutet, dass das Peptid mit der höchsten Affinität zu einem Antikörper oder einem anderen Rezeptor oberflächenexponiert auf dem produzierenden Stamm identifiziert wird. Dabei wird ein entscheidender Vorteil der vorliegenden Erfindung gegenüber für solche Anwendungen verwendeten Phagensystemen (Makowski (1993), Gene 128: 5-11; Kuwabara et al. (1997), Nat. Biotechnol. 15: 74-78) zur Expression von Peptidbibliotheken deutlich. In dem erfindungsgemäßen bakteriellen System erfolgt mit der Identifizierung eines die gewünschte Bindungseigenschaft tragenden Peptids gleichzeitig die Selektion des klonalen Produzenten. Dieser kann unmittelbar vermehrt werden. In einem typischen Beispiel werden klonale Nachkommen des Produzenten zur Reinigung bzw. Isolierung eines Antikörpers oder eines anderen Rezeptors aus einem Molekülgemisch verwendet. Dies geschieht durch Bindung der Rezeptormoleküle mit hoher Affinität an die auf der bakteriellen Oberfläche exponierten Peptidmoleküle, gefolgt von der Abtrennung der nicht gebundenen Moleküle durch Zentrifugation und/oder Filtration und Ablösen der monospezifischen Antikörper oder Rezeptormoleküle von den oberflächenexponierten Peptiden.

Mit der Vermehrung des das gewünschte Peptid oder Protein Oberflächen-exponiert exprimierenden Stammes erfolgt eine Amplifikation des entsprechenden kodierenden Gens, dessen Sequenzanalyse eine eindeutige und einfache Identifizierung und Charakterisierung des Peptids oder Proteins erlaubt. Eine so hergestellte Peptidbibliothek enthält also Fusionproteine, zusammengesetzt aus einem Intimin oder einem Intiminfragment und einem Peptid oder Protein, welches in einem Gram-negativen Bakterium, bevorzugt *E. coli* Oberflächenexponiert produziert wird. In einem typischen Beispiel wird durch Klonierung von an ausgewählten Positionen degenerierten, synthetischen Oligonukleotiden hinter die kodierende Sequenz des Intimins oder Intiminfragments die hohe Varianz der verschiedenen exprimierten Proteine erreicht.

In einer besonders bevorzugten Ausführungsfbrm ermöglicht das erfindungsgemäße Verfahren die Oberflächenexpression und die Variation eines Peptids oder Polypeptids mit einher Affinität zu einem Bindungspartner, einem Liganden, einem Rezeptor, einem Antigen, einem Protein mit enzymatischer Aktivität, einem Antikörper oder einer Antigen-bindenden Domäne eines Antiköpers.

Das erfindungsgemäße Verfahren zur Herstellung einer varianten Population von oberflächenexponierten Peptiden und zur Identifizierung von Bakterien, die Peptide oder Polypeptide mit einer gewünschten Eigenschaft tragen, gliedert sich in folgende Schritte:
1) Herstellung mindestens eines Fusionsgens durch Klonierung der kodierenden Sequenz eines gewünschten Passagiers im durchgehenden Leseraster stromabwärts eines Intimingenes oder Intimingenfragments in mindestens einen Vektor.
2) Variieren des Passagiers durch Klonierung von Passagieren aus einem Gengemisch oder durch ortsgerichtete Mutagenese, z.B. durch Polymerasekeüenreaktion (PCR) unter Verwendung von Oligonukleotiden mit gezielt ausgetauschten Basen, durch Zufallsmutagenese unter Verwendung von Oligonukleotidgemischen mit zufallsmäßig erzeugten Basenabfolgen in ausgewählten Sequenzabschnitten in der PCR, durch error-prone PCR, durch zufallsgesteuerte chemische Mutagenese oder durch Verwendung energiereiches Strahlung.
3) Einbringen des Vektors oder der Vektoren in Wirtsbakterien.
4) Exprimieren des Fusionsgens in den Wirtsbakterien, die dann das Fusionsprotein stabil auf ihrer Oberfläche exprimieren.
5) Kultivieren der Bakterien in Flüssigkultur oder auf Agarplatten zur klonalen Expansion.
6) Selektionieren der Bakterien, die den Passagier mit den gewünschten Eigenschaften tragen und
7) gegebenenfalls die Charakterisierung des selektionierten Passagiers durch Sequenzierung des das Passagierpeptid oder -Polypeptid encodierenden Nukleinsäuresequenzabschnitts und
8) gegebenenfalls die Isolierung und Reinigung eines Bindungspartners für den Passagier mit den optimalen Eigenschaften.

Dabei kann dieses Verfahren mehrmals durchlaufen werden.

Die Isolierung der Bakterien mit einem stabil exponierten Fusionsprotein mit den gewünschten Eigenschaften erfolgt in einer bevorzugten Ausführungsform dieses Verfahrens durch Bindung an einen immobilisierten oder/und markierten Bindungspartner, z.B. einen Matrix-fixierten Bindungspartner, einen Magnetpartikel-markierten Bindungspartner oder einen chromogen oder fluorogen markierten Bindungspartner.

### Bakterielle Oberflächenpräsentation von Proteinfragmenten, Epitopkartierung und Isolierung monospezifischer Antikörper

Jedes Protein trägt viele antigene Determinanten auf seiner Oberfläche. Daher wird die Immunantwort auf ein solches Molekül immer in der Stimulierung vieler B-Lymphozyten und der Produktion ebenso vieler Antikörperspezies bestehen. Für die immunologische Forschung ist die Kenntnis der Aminosäuresequenz solcher Epitope von großer Bedeutung.

Für viele Anwendungen wäre es von Vorteil, wenn man nach der Kartierung von Epitopen direkt große Mengen monospezifischer Antikörper aus dem kartierten Serum gewinnen könnte. Monospezifische Antikörper sind monoklonal in ihren Eigenschaften gleichwertig und werden auch kommerziell vertrieben. Um solche Antikörper zu isolieren, müssen die Peptide, die das Epitop umfassen, an eine Matrix gekoppelt werden. Danach wird das Serum über diese Matrix geleitet, und anschließend werden die spezifisch an das gewünschte Antigen bindenden Antikörper eluiert. In diesem Beispiel der Anwendung der Intimin-basierten bakteriellen Oberflächenpräsentation wurden Epitop-präsentierende *E. coli* Zellen als eine solche Matrix eingesetzt.

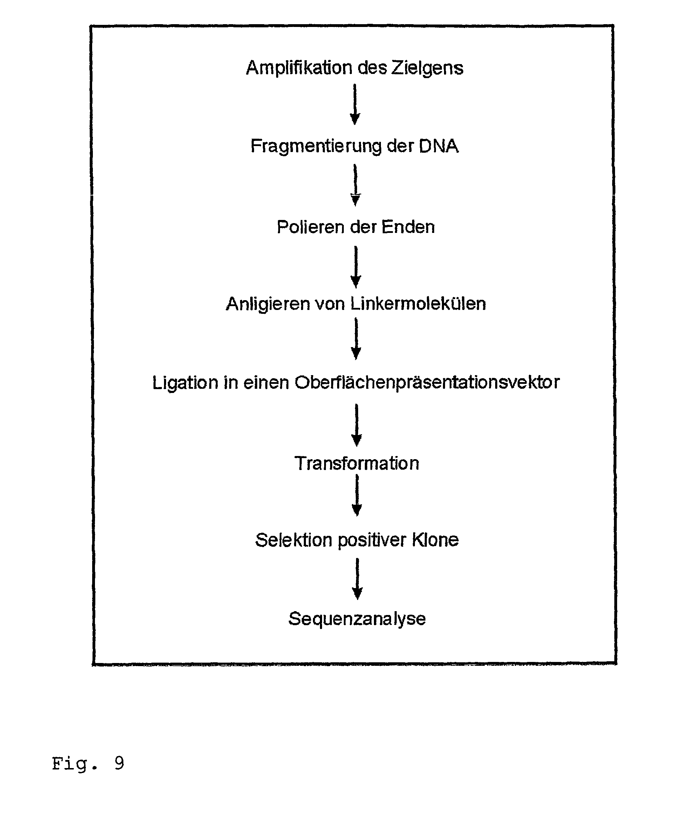

Eine Übersicht zur Vorgehensweise bei der Kartierung von Epitopen mittels Intiminvermittelter Zelloberflächenpräsentation ist in Abb. 9 gezeigt. Die Anwendung dieses Verfahrens ist in Beispiel 2 dargestellt.

### Isolierung von Peptiden mit Affinität zu einem vorgegebenen Zielprotein durch Intimin-basierte Oberflächenpräsentation kombinatorischer Peptidbibliotheken

Um zu überprüfen, ob Intimin-basierte bakterielle Oberflächenpräsentation dazu geeignet ist, aus einem molekularen Repertoire von Oberflächen-präsentierten Peptidvarianten solche mit Affinität zu einem vorgegebenen Zielprotein zu isolieren, wurde eine Bibliothek von Varianten des 28 Aminosäuren umfassen Cystinknoten-Proteins EETI-II generiert. EETI-II ist ein im Kürbisgewächs *Ecballium elaterium* vorkommender Inhibitor von Trypsinproteasen. Dieses Peptid wird durch drei intramolekulare Disulfidbrücken stabilisiert, die eine Reihe von Oberflächenschleifen aufspannen (beschrieben in Wentzel et al. (1999), J. Biol. Chem. 274: 21037-21043). Es wurde eine Bibliothek von EETI-II Varianten generiert, bei der die Lösungsmittel-exponierten Reste zweier Schleifenregionen randomisiert sind. Die experimentelle Durchführung dieses Ausführungsbeispiels ist in Beispiel 3 dargestellt.

### Kurze Beschreibung der Figuren:

Zur Erläuterung der Erfindung dienen ferner die beigefügten Figuren. Im einzelnen zeigen:
- Figur 1:: Schematische Darstellung des Aufbaus eines Intimins. OM: Outer Membrane. D0 bis D3: extrazelluläre Domänenen 1 bis 4. Dem hier bevorzugten Intiminfragment, welches als Träger für Passagierdomänen dient, fehlen die Domänen D2 und D3.
- Figur 2:: Schematische Darstellung des Expressionsvektors pASK-INT-EETI-CK^{Send}. Intimin: Kodierende Sequenz für das Intimingenfragment, etag: Kodierende Sequenz für eine Epitopsequenz (Etag); eeti: kodierende Sequenz für das EETI-II Mikroprotein; cat: Gen für Chloramphenicolacetyltransferase; tetR: Gen für Tet-Repressor; bla: Gen für β-Laktamase.
- Figur 3:: Durchflußzytometrische Analyse der bakteriellen Oberflächenpräsentation eines Intimin-EETI-II Mikroprotein Fusionsproteins.
- Figur 4:: Überlebensraten von *E. coli* Zellen, die vermittels des Intiminmembranankers ein Mikroprotein auf der bakteriellen Zelloberfläche präsentieren.
- Figur 5:: Schematische Darstellung der Kontrolle der Genexpression durch supEvermittelte Translationskontrolle.
- Figur 6a:: Nukleotidsequenz und genetische Organisation des durch die PCR-Primer SupE2-Eco-up und SupE2-Mlu-lo amplifizierten Seqzenzabschnitts des *E. coli* Genoms aus BMH71-18. Die tRNA-codierenden Gene sind durch Fettdruck hervorgehoben.
- Figur 6b:: Schematische Darstellung des Vektors pZA22-MCS1 (Lutz & Bujard (1997), Nucleic Acids. Res. 25:1203) und des aus dem Einbringen des supE-Gens resultierenden Vektors pZA22-supE.. PL-lac-O1: Hybride Operator/Promotorregion aus PL-Operator und lac-Promotor; RBS: Ribosomale Bindungsstelle. MCS-1: Polylinkersequenz. P15A: Replikationsursprung; KanR: Kanamycinresistenzgen.
- Figur 7:: Schematische Darstellung des Vektors pREP4-supE. Plac-supE: Hybride Operator/Promotorregion aus PL-Operator und lac-Promotor (Lutz & Bujard, (1997) Nucleic Acids. Res. 25: 1203) gefolgt von einem das *supE* Gen enthaltenden Sequenzabschnitt. P15A: Replikationsursprung; KanR: Kanamycinresistenzgen, lacI: Gen für den *lac*-Repressor.
- Figur 8:: Regulation der Synthese und Oberflächenpräsentation von Intimin-verankerten Passagierdomänen durch kontrollierte Bereitstellung von supE-tRNA.
- Figur 9:: Vorgehensweise bei der Kartierung linearer Peptidepitope
- Figur 10:: Darstellung der beiden Linkermoleküle resultierend aus der Hybridisierung von 4 Oligonukleotiden, die die entsprechenden Vektorschnittstellen (*Ava*I/*Bam*HI) generieren.
- Figur 11: Lage der identifizierten Epitope in der Proteinsequenz von PMS1
- Figur 12:: A) PMS1_KL6 markiert mit gereinigten Antikörpern gegen Klon 7 (1) und mit gereinigten Antikörpern gegen sein eigenes Epitop (2); B) PMS1_KL7 markiert mit gereinigten Antikörpern gegen Klon 6 (1) und mit gereinigten Antikörpern gegen sein eigenes Epitop (2).
- Figur 13:: Aminosäuresequenz des Repertoires an EETI-II Varianten. Aminoäuren im 1-Buchstabencode. In kursiv sind die beiden Schleifenregionen eingezeichnet, in denen Aminosäurepositionen (X) randomisiert wurden
- Figur 14:: Anreicherung von Mikroproteinen, die anti-β-Laktamase Antikörper binden. Die Zellen wurden jeweils wie im Text beschrieben mit anti-β-Laktamase Antikörper markiert. A) Unselektierte Bibliothek markiert mit anti-β-Laktamase Serum, anti-Kaninchenantikörper (Biotin Konjugat) und Streptavidin-R-Phycoerythrin; in das angelegte Fenster (Fluoreszenzkanal 300-1024) fallen 1,6 % der Zellen. B) zweite Runde der Sortierung (Markierung wie A); C) dritte Runde (Markierung wie A).
- Figur 15:: Aminosäuresequenz der 6 EETI-Varianten die mit dem anti-β-Laktamase Antikörper interagieren; randomisierte Aminosäuren sind grau unterlegt, unterstrichene weisen eine Mutation in der DNA-Sequenz gegenüber dem Wildtyp-Gen auf. Für die hintere Schleifenregion zeichnet sich eine Konsensussequenz für die Bindung an den Anti-β-Laktamase Antikörper ab.

### Beispiele

### Beispiel 1: Genfusion aus einem für ein Passagierprotein und einem für ein IntiminFragment kodierenden Nukleinsäuresequenzabschnitt Vektorkonstruktion und Vergleichsbeispiel

Als Passagier wurde für ein erstes Beispiel das Cystinknotenpolypeptid EETI-II, ein 28 Aminosäurereste umfassender Inhibitor von Trypsinproteasen ausgewählt (wie schon verwendet bei: Wentzel et al. (1999), J. Biol. Chem. 274: 21037-21043; Christmann et al. (1999), Protein Eng. 12: 797-806).

Hierzu wurde das Intimingen eae (Genbank Accession Z11541) aus EHEC O157:H7 Stamm 933 Bakterien mit Hilfe der Oligonukleotide Intiminup und Intiminlo mittels Polymerase Kettenreaktion amplifiziert. Als Templat wurden durch Kochen inaktivierte EHEC O157:H Bakterien eingesetzt. Das Gen wurde amplifiziert mit folgenden Amplifikationsbedingungen: 30 s 94 °C, 30 s 53 °C und 2 min 72 °C, 30 Zyklen.

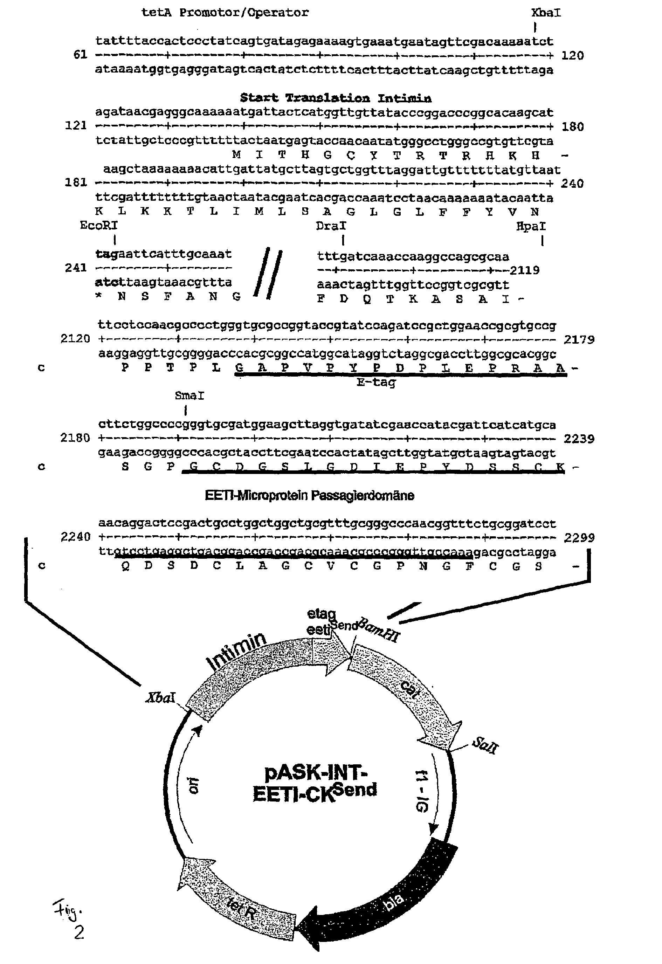

Das Oligonukleotid Intiminup hybridisiert von Nukleotid 69 bis 88 des *eae* Gens. Dieser Sequenzabschnitt beinhaltet das ATG Startcodon, sowie die stromabwärts liegenden 17 Nukleotide. Dieses Oligonukleotid enthält 5' zusätzliche Sequenzen, die eine Klonierung des PCR-Produktes 5'-seitig in die XbaI Schnittstelle des Vektors pASK2ITE-EETI-CK^{Send} (Christmann et al. (1999), Protein Eng. 12: 797-806). Das Oligonukleotid Intilol hybridisiert von Nukleotid 2028 bis 2045 des *eae* Gens und enthält 5'-seitig die codierende Sequenz für eine *Mlu*I Schnittstelle, welche die Klonierung 3' seitig in die EcoRI Schnittstelle des Vektors pAsk21-EETI erlaubt. Das PCR-Produkt enthält somit die kodierende Nukleinsäuresequenz für die Codone 1 bis 659 des Intimins. Im resultierenden Vektor pASK-Inti-EETI ist somit in operativer Verknüpfung mit dem *tetA* Promotor/Operator ein tripartites Fusionsgen lokalisiert, welches besteht aus (a) der codierenden Nukleinsäuresequenz für Intimin von Codon 1 bis 659, (b) der kodierenden Nukleinsäuresequenz für ein 17 Aminosäuren umfassendes Epitop aus dem humanen Gla Protein (Etag; Kiefer et al. (1990), Nucleic Acids Res. 18: 1909) und (c) der kodierenden Nukleinsäuresequenz für das Cystinknotenpolypeptid EETI-CK^{Send} (Christmann et al. (1999), Protein Eng. 12: 797-806); Abb. 2).

Durch Klonierung eines PCR-Fragments wurde das CAG-Codon Nummer #35 in Intimin gegen ein TAG-Stopcodon ausgetauscht.

Um das Expressionslevel mit bisherigen Verfahren zur Oberflächenpräsentation vergleichen zu können, wurde ein zweites Plasmid herangezogen (pASK21TE-EETI-CK^{Send}), bei dem die Intiminsequenz durch ein verkürztes OmpA-Fragment ersetzt ist (Christmann et al. (1999), Protein Eng. 12: 797-806).

Als Bakterienstamm für die Genexpression wurde der Stamm BMH71-18 verwendet (Christmann et al. (1999), Protein Eng. 12: 797-806).

### BMH71-18: [F' lacl^{q} /acZΔM15, proA⁺B⁺; Δ(lac-proAB), supE, thi]

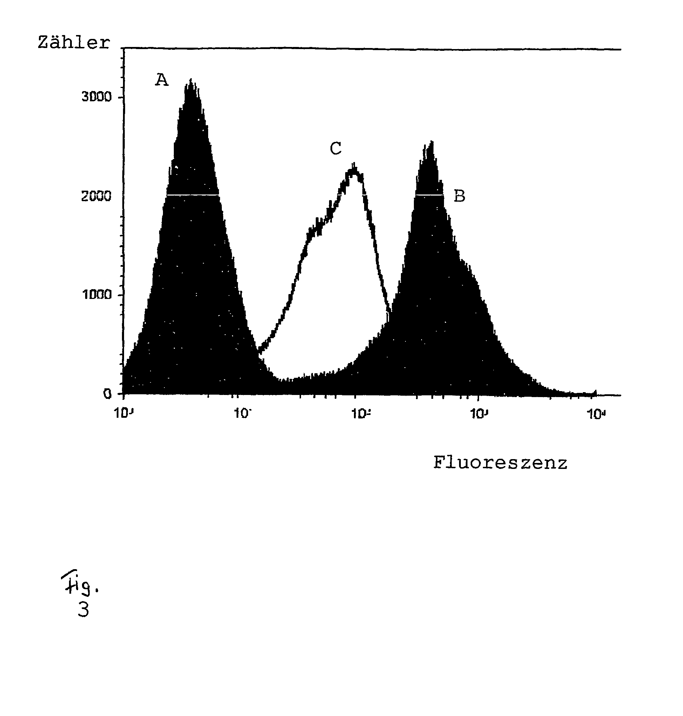

Die transformierten Zellen wurden bei 37°C bis zu einer optischen Dichte von 0,2 bis 0,4 anwachsen lassen. Die Expression des Fusiongens wurde durch Zugabe von 0,2 µg/ml Anhydrotetrazyklin induziert. Die Oberflächenpräsentation wurde über die Bindung eines monoklonalen Anti-Etag-Antikörpers an die Oberfläche der Zellen über eine indirekte Fluoreszenzmarkierung nachgewiesen. Hierfür wurden die Zellen nach einer Stunde Inkubation abzentrifugiert und genau wie bei Christmann et al. (1999), Protein Eng. 12: 797-806) beschrieben sukzessive mit monoklonalem Anti-E-tag Antikörper, biotinyliertem Anti-Maus-Antikörper und mit Streptavidi, Phycoerythrin-Konjugat inkubiert. Die markierten Zellen wurden im Durchflußzytometer vermessen (Abb. 3). Das FACS-Diagramm zeigt die Fluoreszenz von Zellen, die kein Fusionsprotein produzieren (Negativkontrolle, A), solchen die das Intimin-Etag-EETI Fusionsprotein produzieren (B) und zum Vergleich von solchen Zellen, die mit einem Plasmid transformiert sind, bei dem der Intimin-Membrananker durch einen dem bisherigen Sder Technik entsprechenden 1pp-OmpA Membrananker ersetzt ist (C).

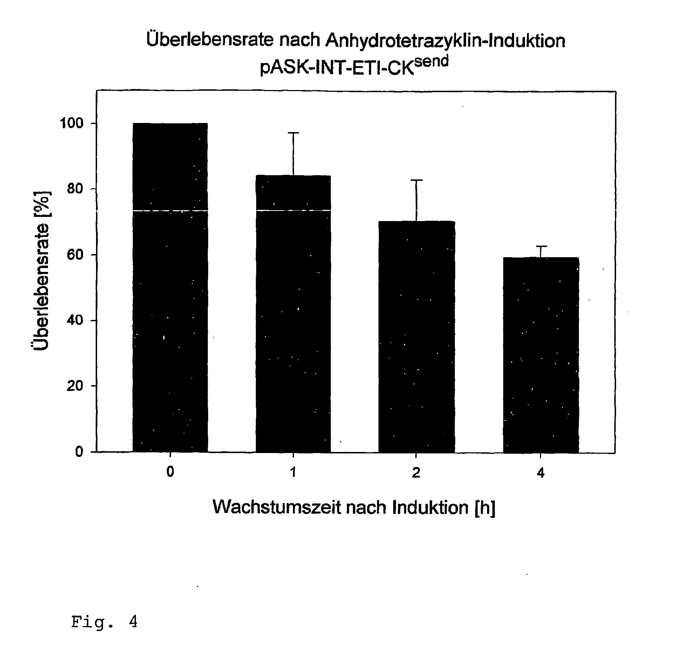

Wie aus dieser Abbildung zu entnehmen ist, zeigen die Zellen, bei denen eine Intimin-vermittelte Präsentation des Fusionspartners stattfindet eine ca. 10-fach höhere Fluoreszenz als die Zellen mit herkömmlichem Membrananker. **Dieser Befund zeigte überraschenderweise, dass durch Einsatz des Intimin-Membranankers ca. 10 mal mehr Moleküle des Passagierproteins auf der Bakterienoberfläche zur Verfügung stehen. Darüberhinaus wird die Überlebensfähigkeit der Zellen,** die bei bisher beschriebenen Verfahren der Überpression von membranverankerten Passagierproteinen stark abnimmt, **durch die Überproduktion des Fusionsproteins aus Intiminfragment und Passagierdomäne nicht nennenswert beeinträchtigt.** Nachfolgend ist die Überlebensrate der Zellen vor und 1,2 und 4 Stunden nach Induktion der Expressions des Fusionsproteins gezeigt (Abb. 4).

### Kombinierte Verwendung von supE tRNA in Wirtsorganismen und amber Stopcodon im Intiminfragment

Durch Klonierung eines PCR-Fragments wurde das CAG-Codon Nummer #35 in Intimin gegen ein TAG-Stopcodon ausgetauscht. Bei Verwendung eines *amber* Suppressorstammes wird dieses Stopcodon überlesen und an dieser Stelle ein Glutaminrest eingebaut. Da die Effizienz der *amber* Suppression niedrig ist, werden dadurch weniger Moleküle synthetisiert, als bei Abwesenheit des Stopcodons. Dadurch wird sichergestellt, dass die Zahl der oberflächenpräsentierten Moleküle in einem für die Zelle tolerablen Umfang bleibt (siehe hierzu Christmann et al. (1999), Protein Eng. 12: 797-806). Die Nukleotidsequenz des 5'-terminalen und des '3'-terminalen Teils des Intimingens im Expressionsvektors pASK-INT-EETI-CK^{send} ist in Abb. 2 dargestellt, das *amber* Stopcodon #35 ist dabei durch Fettdruck hervorgehoben.

Um das *supE* zu erhalten, wurde der *amber* Suppressor Stamm BMH71-18 als PCR Templat verwendet.

### BMH71-18: [F' lacl^{q} lacZΔM15, proA⁺B⁺; Δ(lac-proAB), supE, thi]

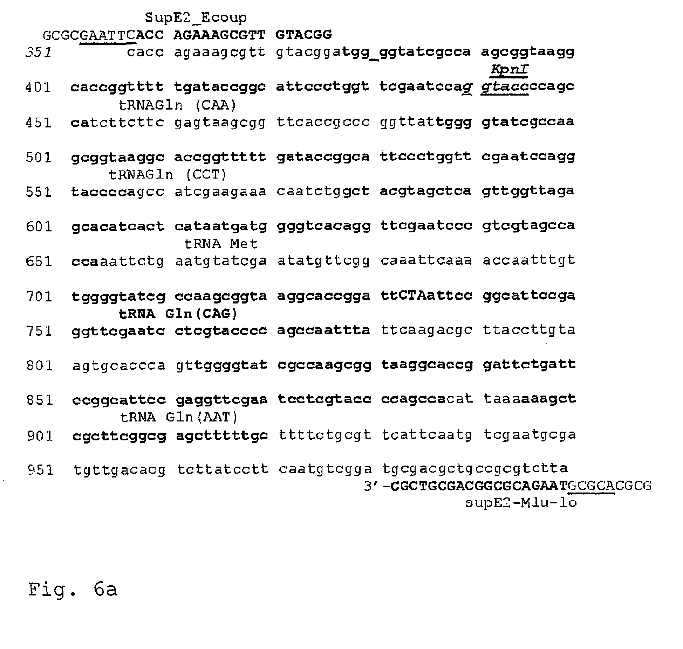

Das Gen wurde unter Verwendung der Primer SupE2-Eco-up und SupE2-Mlu-lo unter Standardbedingungen aus dem Genom amplifiziert. Die Sequenz des Genomabschnitts und die Primer sind in Abbildung 6a eingezeichnet.

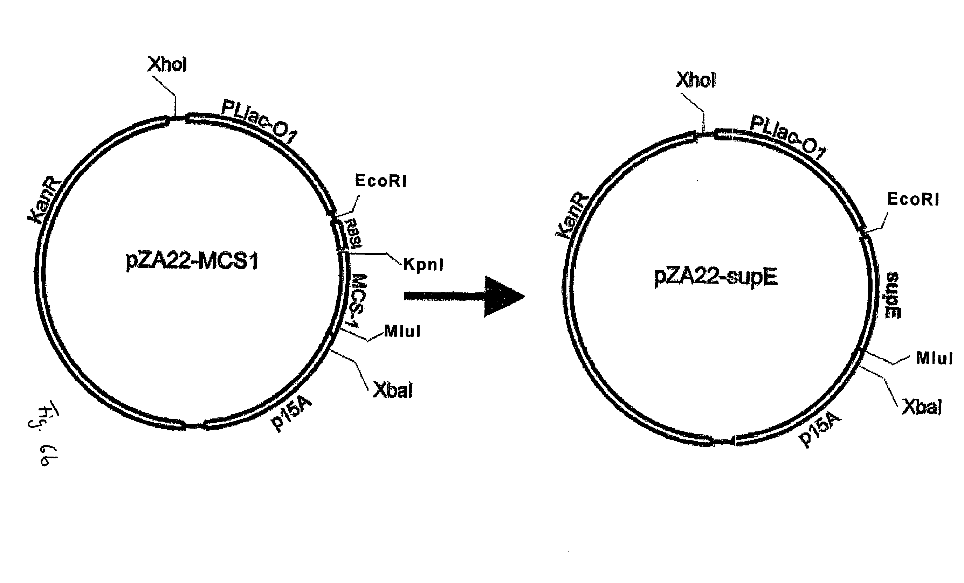

Zur Amplifikation wurden die beiden PCR-Primer supE2-Eco-up und SupE2-Mlu-lo verwendet. Das *supE*-Gen liegt in einem Cluster von tRNA-Genen, die polycistronisch transkribiert und anschließend durch spezifische RNAsen prozessiert und freigesetzt werden. Mit diesen Oligonukleotiden wird das *supE*-Gen und jeweils stromaufwärts und stromabwärts mindestens ein zusätzliches tRNA-Gen amplifiziert. Außerdem führen die Primer eine *Eco*RI- und eine *Mlu*I Schnittstelle ein. Der resultierende DNA-Abschnitt wurde mit *Eco*RI und *Mlu*I gespalten und in den mit EcoRI und MluI gespaltenen Vektor PZA22-MCS1 (Lutz & Bujard (1997), Nucleic Acids. Res. 25:1203) gebracht, wie in Abb. 6b dargestellt.

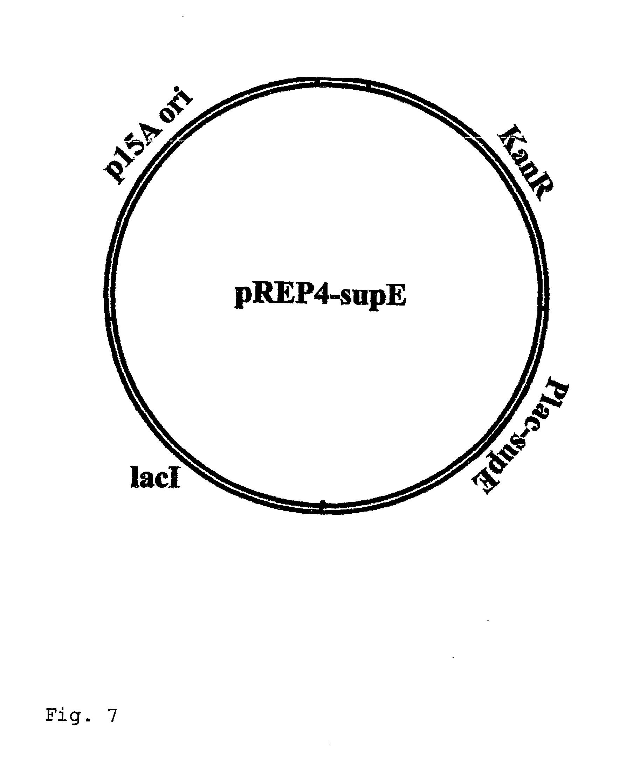

In diesem Vektor befindet sich der das supE-Gen enkodierende Sequenzabschnitt nunmehr unter der Kontrolle des mit IPTG induzierbaren PLlacO-1 Promotor/Operators MCS (Lutz &Bujard, Nucleic Acids. Res. 1997, 25:1203). Der Promotor/Operator und der stromabwärts liegende *supE*-Gensequenzabschnitt wurde durch Spaltung des resultierenden Plasmids mit *Xho*I und *Xba*I entnommen. Die Enden wurden mit T4-Polymerase in Gegenwart von dATP, dGTP, dCTP und dTTP aufgefüllt. Das resultierende Fragment wurde in den mit *Sma*I gespaltenen Vektor pRep4 (Qiagen) eingesetzt. Eine schematische Karte des resultierenden Vektors ist in Abb. 7 gezeigt.

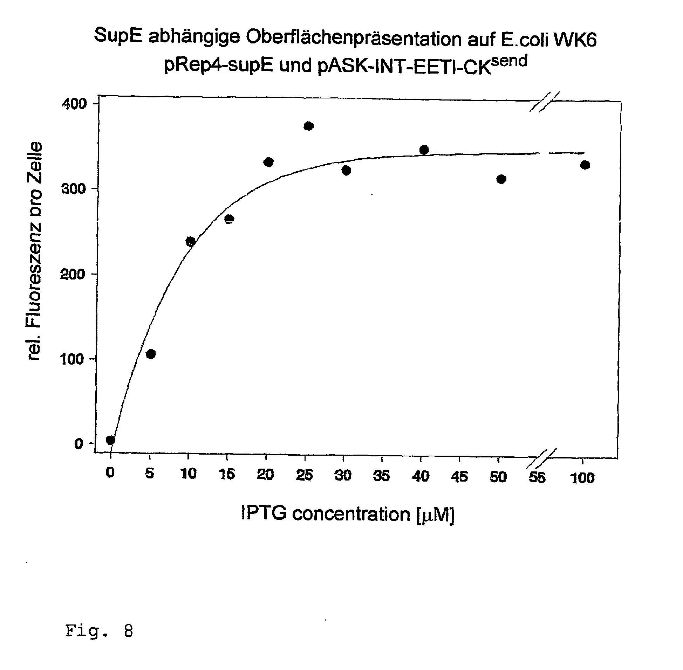

Von pREP4-supE kann durch IPTG-Induktion die Transkription des *supE*-Gens und damit die Bildung von supE-tRNA induziert und durch Variation der Menge an Induktor auch gesteuert werden. Zum Nachweis der Regulierbarkeit der Expression oberflächenpräsentierter Proteine wurde das Plasmid pREP4-supE in den *E. coli* Stamm WK6 [(*lac-pro*AB), *thi, rpsL, nal^{T};* F'*lac*I^{q}, lacZM15, *pro*A+B+] eingebracht. Dieser Stamm besitzt kein chromosomal kodiertes *supE*-Gen. Zusätzlich wurde der Stamm mit dem Expressionsplasmid pASK-INT-EETI-CK^{Send} (s.o.) transformiert. Der nunmehr mit zwei Plasmiden transformierte E. coli Stamm wurde in Parallelansätzen in Gegenwart steigender Mengen an IPTG angezogen (0, 5, 10, 15, 20, 30, 40, 50, 1000 µM). Bei Erreichen einer Optischen Dichte von 0,2 wurde 0,2 µg/ml Anhydrazyklin zur Induktion der Transkription des Intimingens zugegeben. Nach einer Stunde Induktion wurden die Zellen geerntet. Die Zellen wurden sukzessive mit Anti-Etag Antikörper, biotinyliertem Ziegen-Anti-Maus Antikörper und schließlich mit Phycoerythrin gekoppeltem Streptavidin markiert (siehe oben). Die pro Zelle dadurch hervorgerufene Fluoreszenz ist dabei proportional zur Anzahl der auf der Oberfläche der Zelle präsentierten Intimin-Fusionsproteine. Die zelluläre Fluoreszenz wurde durchflußcytometrisch bestimmt. Wie aus Abb. 8 hervorgeht, ist die Intensität der Fluoreszenz pro Zelle - und damit die Anzahl der präsentierten Moleküle - direkt mit der Menge an IPTG-Induktor korreliert und kann in weiten Bereichen moduliert werden.

### Beispiel 2: Bakterielle Oberflächenpräsentation von Proteinfragmenten, Epitopkartierung und Isolierung monospezifischer Antikörper

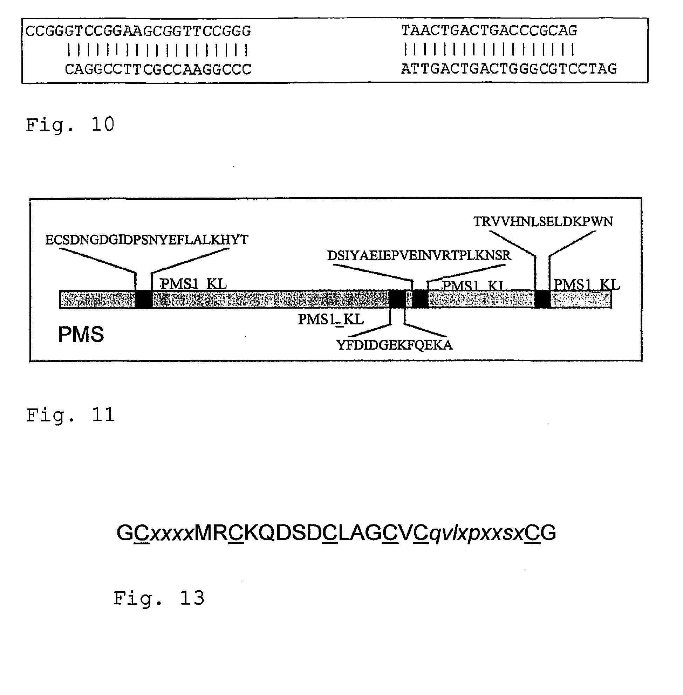

Das Gen für PMS1 aus der Hefe *S. cerevisiae* (Genbank Accession Nr. M29688) wurde als Modellantigen verwendet. Das Gen (2.7 kB) wurde mit Hilfe der PCR-Primer PMS1up und PMS1lo aus *S. cerevisiae* amplifiziert. Die DNA wurde mit dem Nukleotrap-PCR Kit (Machery und Nagel) aufgereinigt. Der DNAse I Verdau wurde in vier verschiedenen Ansätzen mit 1,8*10⁻², 2,7*10⁻², 3,6*10⁻² und 0,18 u DNAseI in Gegenwart von 2mM MnCl₂ durchgeführt. Pro Ansatz wurden 5 µg DNA eingesetzt. Die Restriktionsspaltung wurde nach 10 min bei RT durch Zugabe von 500 mM EDTA abgestoppt. Die entstandenen Fragmente wurden nach Anfärben mit Ethidiumbromid auf einem 12,5 %igen Polycrylamidgel aufgetrennt. Es wurden Fragmente im Größenbereich von 40-100 bp ausgeschnitten. Anschließend wurden die Fragmente aus dem Gel über Nacht in TBE-Puffer durch Diffusion eluiert. Anschließend wurde möglicherweise noch vorhandene DNAse I mittels Phenol/Chloroform Extraktion und Ethanolfällung der DNA entfernt. Daraufhin wurden überhängende Enden mit T4-DNA-Polymerase in Gegenwart von dATP, dCTP, dGTP und dTTP aufgefüllt. Dann wurden Oligonukleotidhybridlinker (s. Abb. 10) anligiert (2,5 molarer Überschuß 16 h 15 °C) und die Ligationsprodukte erneut über ein 12,5 %iges Acrylamidgel aufgereinigt.

Die Elutionsbedingungen waren die gleichen wie bei der Aufreinigung der Genfragmente. Anschließend wurde die DNA erneut Phenol/Chloroform extrahiert und gefällt. Der Klonierungsvektor pASK-INT-EETI-CK^{Send} wurde mit *Ava*I/*Bam*HI gespalten und durch Sucrosegradientendichtezentrifugation gereinigt (Kolmar, H. & Fritz, H.-J. (1995). Oligonucleotide-directed mutagenesis with single-stranded cloning vectors. In: DNA Cloning 1: A practical approach. D. Glover, B. D. Hames (Eds.). IRL Press, Oxford, pp. 193-224). Insgesamt wurden pro Genbank 230 ng gespaltener Vektor mit der fragmentierten DNA ligiert und damit elektrokompetente BMH 71-18 Zellen transformiert. Es wurde ein Repertoire von 7,2*10⁴ unabhängigen Klonen erhalten. Die Population von Bakterienzellen wurde nach Induktion der Expression des Intimin-Fusionsgens durch Zugabe von Anhydrotetrazyklin mit polyklonalem Anti-PMS1 Kaninchenserum inkubiert. Die spezifische Bindung von Antikörpern an Epitop-tragenden Zellen wurde durch Markierung mit biotinyliertem Anti-Kaninchen-Antikörper und Inkubation mit Streptavidin-Phycoerythrin Konjugat nachgewiesen. Fluoreszenz-markierte Zellen wurde durch FACS sortiert und mit Hilfe eines MoFlo Cellsorters (Cytomation) einzeln auf einer Agarplatte abgelegt. Von einer Anzahl von Klonen wurde DNA präpariert und die Nukleotidsequenz von vier klonierten PMS1-Genfragmenten bestimmt. Die DNA-Sequenz der Klonen umfaßte die Basen 259-330 (PMS1_KL3), 1630-1672 (PMS1_KL6), 1786-1856 (PMS1_KL7) und 2590-2639 (PMS1_KL10) des *pms1* Gens (insgesamt 2715 bp). In Abb. 11 ist die Position dieser Epitope in der Peptidsequenz des PMS1-Proteins, sowie die translatierte Aminosäuresequenz des klonierten PMS1 Sequenzabschnitts angegeben.

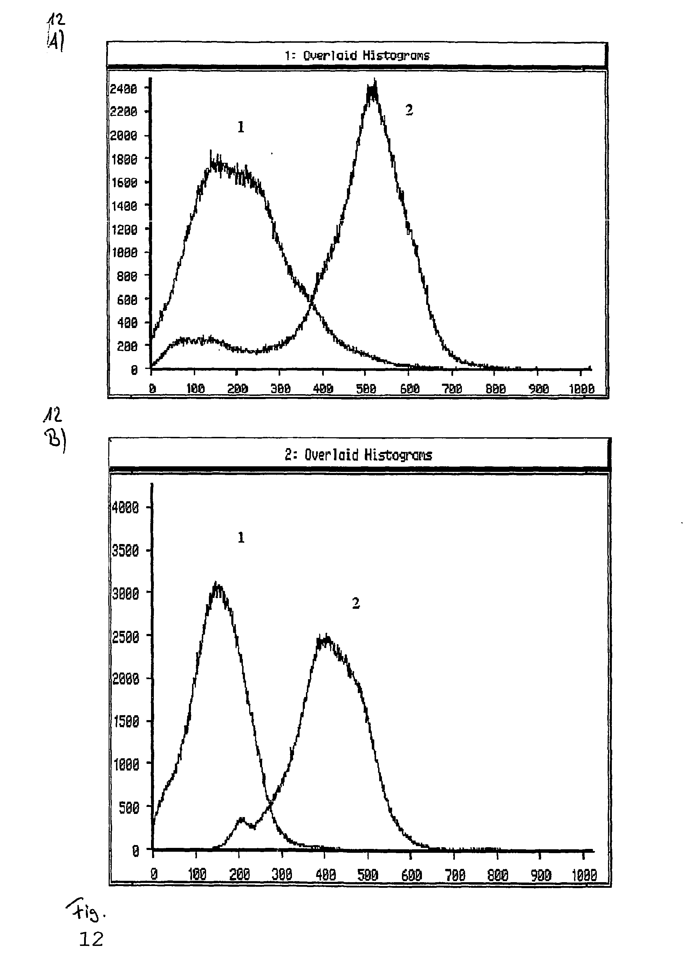

Von den Klonen PMS_KL6 und PMS_KL7 wurden Kulturen im 50 ml Maßstab angezogen. Nach einer Stunde Induktion durch Zugabe von Anhydrotetrazyklin (0,2 µg/ml) bei einer optischen Dichte von 0,2 wurden die Zellen pelletiert, mit PBS-Puffer gewaschen und in 500 µl PBS resuspendiert. Anschließend wurde zu diesen Bakterien je 200 µl PMS1 Serum gegeben und die Suspension für 45 min auf Eis inkubiert. Anschließend wurden die Zellen pelletiert und der Überstand verworfen. Das Zellpellet wurde in 200 µl Glycin/NaCl (0,2 M Glycin, 0,14 M NaCl, pH 2,0) resuspendiert und für 45 min auf Eis inkubiert. Nach der Inkubation wurden die Bakterien abzentrifugiert und der Überstand durch Zugabe von 100 µl 1 M Tris/HCl pH9 alkalisiert. Mit den so gewonnenen monospezifischen Antikörpern wurden die Zellen von Klon KL6 bzw. KL7 inkubiert (s. Abb. 12).

Damit ist gezeigt, dass mit diesem Verfahren monospezifische Antikörper isoliert werden können.

### Beispiel 3: Isolierung von Peptiden mit Affinität zu einem vorgegebenen Zielprotein durch Intimin-basierte Oberflächenpräsentation kombinatorischer Peptidbibliotheken

Es wurde eine Bibliothek von Varianten des 28 Aminosäuren umfassen Cystinknoten-Proteins EETI-II generiert. EETI-II ist ein im Kürbisgewächs *Ecballium elaterium* vorkommender Inhibitor von Trypsinproteasen. Dieses Peptid wird durch drei intramolekulare Disulfidbrücken stabilisiert, die eine Reihe von Oberflächenschleifen aufspannen (beschrieben in Wentzel et al. (1999), J. Biol. Chem. 274: 21037-21043). Es wurde eine Bibliothek von EETI-II Varianten generiert, bei der die Lösungsmittel-exponierten Reste zweier Schleifenregionen randomisiert sind. Die Aminosäuresequenz des EETI-II Repertoires ist:
GCXXXXMRCKQDSDCLAGCVCQVLXPXXSXCG

### (Aminoäuren im 1-Buchstabencode. In kursiv sind die beiden Schleifenregionen eingezeichnet, in denen Aminosäurepositionen (X) randomisiert wurden)

Die randomisierten *eeti*-Gene wurden mittels PCR generiert. Als Templat wurde ein *eeti-ck^{send}* Gen (siehe oben) eingesetzt. Dieses wurde mit Hilfe der denegerierten Primer eti_4+4up und eti_4+41o amplifiziert. Die Randomisierung der entsprechenden Codone in der Primersequenz erfolgte nach dem Muster NNS, wobei N jedes der vier Nucleotide und S G oder C darstellt. Durch diese Wahl wurden die Stopcodone *ochre* (TAA)und *opal* (TGA) ausgeschlossen und die Zahl der möglichen Codone von 64 auf 32, die aber noch für alle Aminosäuren kodieren, reduziert. Das Gemisch resultierender PCR-Produkte wurde mit *Ava*I und *Bam*HI geschnitten und mit dem *Ava*I/*Bam*HI gespaltenen pASK-INT-EETI-CK^{send} Vektorfragment ligiert.

Als Klonierungsvektor diente der pASK-INT-EETI-CK^{send} (s. Abb. 8). Dieser Vektor wurde mit *Ava*I und *Bam*HI geschnitten. Anschließend wurde das Vektorfragment mittels Sucrosegradientendichtezentrifugation von der Vektor DNA abgetrennt. Die durch PCR mit degenerierten Primern generierten *eeti*4+4 Gene wurden mit dem gereinigten Vektor ligiert und mit diesem Ansatz der E. coli Stamm DH5α (Hanahn, D. (1983), J. Mol. Biol. 166: 557-580) transformiert. Es wurden 16 Transformationen parallel durchgeführt. Alle Transformanten wurden auf Chloramphenicol-haltige Selektivplatten (25 µg/ml) ausgestrichen. Nach Inkubation für ca. 20 Stunden bei 37 °C wurden die Kolonien abgeschwemmt und die Zellen nach Zugabe von DMSO (7% v/v) in Aliquots bei -80 °C gelagert. Insgesamt wurden so 2*10⁷ unabhängige Klone erzeugt.

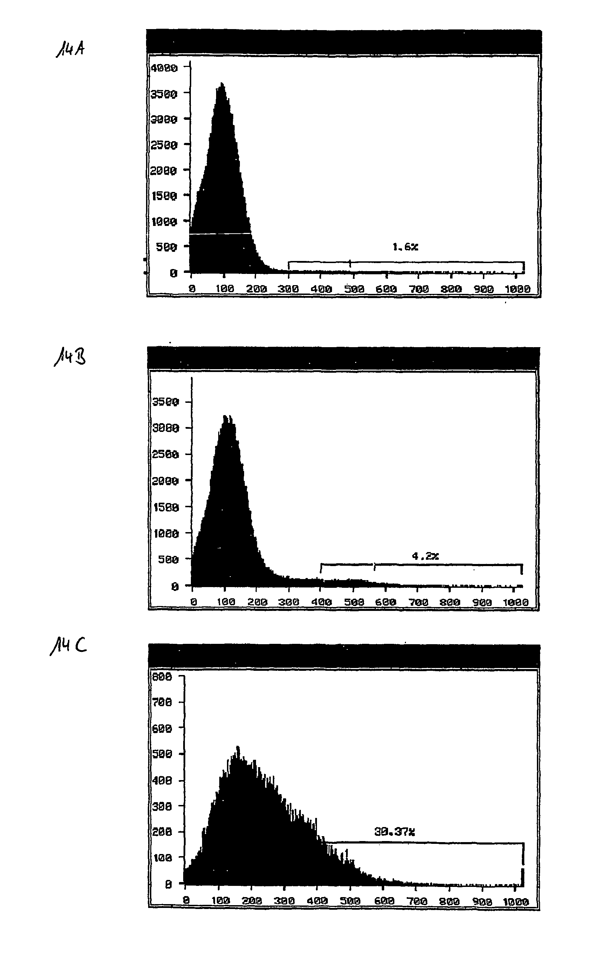

Zur Isolierung von Mikroproteinen mit Affinität zu Anti-β-Laktamaseantikörpern wurde eine Flüssigkultur von 50 ml mit 10⁹ Zellen der gelagerten Bibliothek angeimpft. Bei Erreichen einer optischen Dichte von 0,4 wurde Anhydrotetrazyklin zur Induktion der Genexpression zugegeben (0,2 µg/ml). Nach der Induktion wurden die Zellen Fluoreszenz-markiert. Dazu wurden sie zuerst für 10 min Anti-β-Laktamase-Antikörper inkubiert, anschließend mit PBS gewaschen, um nicht gebundenes Protein zu entfernen. Anschließend wurden die Zellen mit biotinyliertem Anti-Kaninchenantikörper und dann mit Streptavidin-gekoppeltem R-Phycoerythrin inkubiert. Nach der Markierung wurden die Zellen im Cellsorter analysiert und fluoreszierende Zellen aussortiert. Als fluoreszierend wurden solche Bakterien definiert, die in einen Fluoreszenzkanal zwischen 300-1024 fielen (s. Abb. 14).

Aussortierte Zellen wurden auf Chloramophenicol-haltige (25 µg/ml) Agarplatten plattiert und über Nacht bei 37 °C inkubiert. Am darauffolgenden Tag wurden die Kolonien abgeschwemmt und als DMSO-Kultur bei -80 °C gelagert. Ein Aliquot wurde verwendet, um eine frische 50 ml Kultur zu beimpfen.

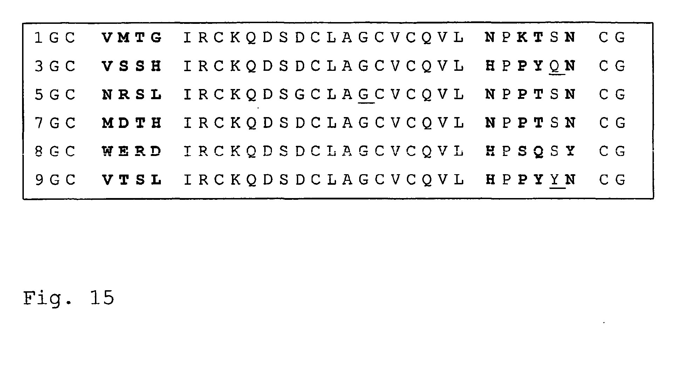

In der ersten Runde wurden insgesamt 2*10⁸ Zellen analysiert und solche, die in den entsprechenden Fluoreszenzkanal (300-1024) fielen, wurden aussortiert. In der zweiten Runde wurden Zellen, die in diesen Fluoreszenzkanal fielen (insgesamt 4,2%), aussortiert und anschließend noch zweimal direkt resortiert. Diese Zellen (5*10⁵ Ereignisse) wurden ausplattiert und gingen am nächsten Tag in die dritte Runde. Hier wurde ein signifikanter Anstieg von Bakterien, die in den selektierten Bereich fallen, registriert (30 %). Die Zellen wurden erneut sortiert und plattiert, um am darauffolgenden Tag Einzelklone zu analysieren. 15 von 20 Einzelklonen zeigten eine Interaktion mit anti-β-Laktamase Antikörper. Aus 6 diesen Klonen wurde Plasmid-DNA isoliert und die Nukleotidsequenz des Gens für die EETI-CK-Variante bestimmt (Abb. 15).

Im Rahmen dieser Erfindung verwendbare Proteinsequenzen aus der Familie der Intimine sind an folgenden Stellen in der Literatur beschrieben:
1.) Gamma Intimin (Escherichia coli), NCBI GI 3941710, NCBI GI 3941712, NCBI GI 3941714, McGraw, E. A. et al. in "Molecular evolution and mosaic structure of alpha, beta, intimins of pathogenic Escherichia coli", Mol. Biol. Evol. 16(1) 12-22 (1999)
2.) Intimin (attaching and effacing protein, eae Protein), NCBI GI 1169452, Yu, J1 and Kaper, J.B. in "Cloning and characterization of the eae gene of enterohae. Escherichia coli O157:H7" Mol. Microbiol. 6(3), 411-417 (1992)
3.) eae Gen, NCBI GI 384173, Beebakhee, G. et al. in "Cloning and nucleotide sequence of the eae gene homologue enterohemorrhagic Escherichia coli serotype O157/H7," FEMS Microbiol. Lett. 91(1), 63 - 68 (1992)
4.) Intimin (Escherichia coli), NCBI GI 2565325, Voss, E. et al. in "Translocated intimin receptors (Tir) of shiga-toxigenic E. coli isolates belonging to serogroups O26, O111, and O157 with sera from patients with hemolytic-uremic syndrome and marked sequence heterogeneity", Infect. Immun. 66 (11), 5580 - 5586 (1998)
5.) Intimin (Escherichia coli) NCBI GI 2865299, Elliott, S.J. et al. in "The complete sequence of the locus of enterocyte effacement from enteropathogenic Escherichia coli E2348/69", Mol. Microbiol. 28 (1), 1-4 (1998)
6.) Beta Intimin (Escherichia coli), NCBI GI 3941718, McGraw et al.a.a.O:
7.) Intimin (Escherichia coli) NCBI GI 2739264, Deibel, D. et al. in "EspE, a novel secreted protein of attaching and effacing is directly translocated into infected host cells, where as a tyrosine-phosphorylated 90 kDa protein", Mol. Microbiol. 28 (3), 463-474 (1998)
8.) Intimin (Escherichia coli) NCBI GI 4388530; 9.) Intimin (Escherichia coli) NCBI GI 4388530; 10.) Intimin type epsilon (Escherichia coli) NCBI GI 6683770;
11.) Intimin NCBI GI 1947048; 12.) Intimin NCBI GI 4106360; 13.)Intimin (Escherichia coli) NCBI GI 6649538; 14.) Intimin (Escherichia coli) NCBI GI 2809548; 15.) Intimin (Escherichia coli) NCBI GI 7384863; 16.) Shares homology with the enteropathogenic E.coli (EPEC) coli attaching and effacing) Gene, putative NCBI GI 304362; 17.) Intimin (Escherichia coli) NCBI GI 7384863

### Verwendete Oligonukleotide

Intiminup:
   5'-GCGCTCTAGATAACGAGGGCAAAAAATGATTACTCATGGTTGTTATAC-3'
Intilo1:
   5'-GCGCCAATTGCGCTGGCCTTGGTTTGATC-3'
SupE2_Ecoup
   5'-GCGCGAATTCACC AGAAAGCGTT GTACGG-3'
SupE2-Mlu-lo
   5'-GCGCACGCGTAAGACGCGGCAGCGTCGC-3'
PMS1up
   5'-GCGATGTTTCACCACATCG-3'
PMS1lo
   5'-TCATATTTCGTAATCCTTC-3'
eti-4+4up:
   5'-GACGCCCGGGTGCNNSNNSNNSNNSATCCGTTGCAAACAGGACTCCG-3'
eti-4+41o:
   5'-GACGCCCGGGTGCNNSNNSNNSNNSATCCGTTGCAAACAGGACTCCG-3'
ML/Ko1

## Patentansprüche

1. Verfahren zur Präsentation von Peptiden und Proteinen auf der Oberfläche von Wirtsbakterien, wobei man (a) ein gramnegatives Wirtsbakterium bereitstellt, das transformiert ist mit einem Vektor, auf dem in operativer Verknüpfung mit einem exogen induzierbaren Promotor eine fusionierte Nukleinsäuresequenz lokalisiert ist, welche: (i) einen Sequenzabschnitt, der ein im carboxyterminalen Bereich um wenigstens die D3-Domäne verkürztes Intimin kodiert, als Verankerungsdomäne und (ii) einen für das zu präsentierende Passagierpeptid bzw. Passagierpolypeptid/protein kodierenden Nukleinsäuresequenzabschnitt aufweist, und (b) das Wirtsbakterium unter Bedingungen kultiviert, bei denen eine Expression der fusionierten Nukleinsäuresequenz und eine Präsentation des von dem Nukleinsäure-Sequenzabschnitt (ii) kodierten Peptids/Polypeptids/Proteins an der Oberfläche des Wirtsbakterium erfolgt, wobei der Nukleinsäure-Sequenzabschnitt (ii) gegenüber dem für die Intimin-Membran-Verankerungsdomäne kodierenden Nukleinsäure-Sequenzabschnitt heterolog ist.

2. Verfahren nach Anspruch 1, **dadurch gekennzeichnet, dass** das verkürzte Intimin im carboxylterminalen Bereich von 280 Aminosäuren um wenigstens eine weitere der Intimin-Domänen D0, D1, D2 verkürzt ist.

3. Verfahren nach Anspruch 1, **dadurch gekennzeichnet, dass** die verwendete Intimin-Verankerungsdomäne in der äußeren Bakterienmembran aus der Gattung der Enterobacteriaceae abgeleitet wurde und in einem Wirtsbakterium einer Gattung der Enterobacteriaceae verwendet wird.

4. Verfahren nach Anspruch 3, **dadurch gekennzeichnet, dass** als Verankerungsdomäne ein Fragment des Intimins aus enterohämorrhagischen E. coli oder eine Variante davon verwendet wird.

5. Verfahren nach Anspruch 4, **dadurch gekennzeichnet, dass** eine carboxylterminal verkürzte Variante des EaeA-Intimins aus enterohämorrhagischen Eschericia coli O157:H7 als Verankerungsdomäne verwendet wird.

6. Verfahren nach Anspruch 5, **dadurch gekennzeichnet, dass** die verkürzte Variante des EaeA-Intimins die Aminosäuren 1 bis 659 beinhaltet.

7. Verfahren nach Anspruch 5, **dadurch gekennzeichnet, dass** die verkürzte Variante des EaeA-Intimins die Aminosäuren 1 bis 753 beinhaltet.

8. Verfahren nach Anspruch 1, bei dem die Expression des Fusionsgens aus dem für das verkürzte Intimin und dem für das Passagierprotein kodierenden Nukleinsäuresequenzabschnitt regulierbar ist, wobei (i) ein für ein Glutamin kodierendes Codon des für das verkürzte Intimin kodierenden Nukleinsäuresequenzabschnitts durch ein amber Stopcodon (TAG) ersetzt ist und (ii) ein Escherichia coli Wirtsstamm verwendet wird, in dem ein Translation der mRNA des Fusionsgens durch Bereitstellung einer regulierbaren Menge an Suppresor-tRNA erfolgt, die ein Überlesen des Stopcodons bei der Translation ermöglicht.

9. Verfahren nach Anspruch 8, bei dem die Regulation der Expression des Gens für die amber Suppressor tRNA **dadurch** stattfindet, dass dieses unter Kontrolle eines in seiner Transkriptionsrate regulierbaren Promotors gesetzt wird.

10. Verfahren nach Anspruch 9, bei der der in seiner Transkriptionsrate regulierbare Promotor der PL1ac-Promotor ist.

11. Verfahren zur Herstellung einer varianten Population von oberflächenexponierten Peptiden/Polypeptiden/Proteinen und zur Identifizierung von Bakterien, die Peptide/Polypeptide/Proteine mit einer jeweils gewünschten Eigenschaft tragen, wobei das Verfahren folgende Schritte aufweist: (i) Herstellen eines oder mehrerer Fusionsgene oder Fusionsfragmente durch Klonierung der kodierenden Sequenz eines gewünschten Passagiers im durchgehenden Leseraster mit der kodierenden Sequenz des im carboxylterminalen Bereich um wenigstens die D3-Domäne verkürzten Intimingens in mindestens einen Expressionsvektor; (ii) Variieren des Passagierpeptids durch eine der Methoden ausgewählt aus: gezielte, ortsgerichtete Mutagenese, z.B. durch Polymerasekettenreaktion (PCR) unter Verwendung von Oligonukleotiden mit gezielt ausgetauschten Basen, durch Zufallsmutagenese unter Verwendung von Oligonukleotidgemischen mit zufallsmäßig erzeugten Basenabfolgen in ausgewählten Sequenzabschnitten in der PCR, durch error-prone PCR, zufallsgesteuerte chemische oder strahlungsgenerierte Mutagenese, (iii) Einbringen des wenigstens einen Vektors in Wirtsbakterien, die den Passagier stabil auf ihrer Oberfläche präsentieren, (iv) Exprimieren des Fusionsgens in den Wirtsbakterien, (v) Kultivierung der Bakterien zur Produktion eines stabil obeflächenexponiert präsentierten Passagiers, und (vi) selektive Anreicherung der Bakterien, die den Passagier mit den gewünschten Eigenschaften auf der Oberfläche tragen.

12. Verfahren nach Anspruch 11, bei dem die Identifizierung des Bakteriums, das einen Passagier mit einer gewünschten Bindungsaffinität oberflächenexponiert präsentiert, durch Bindung an einen immobilisierten oder/und markierten Bindungspartner erfolgt.

13. Verfahren nach Anspruch 11, bei dem eine Population von Bakterien, welche einen Passagier mit einer Bindungsaffinität zu einem Bindungspartner oberflächenexponiert tragen, als Matrix zur Affinitäts-chromatographischen Reinigung des Bindungspartners aus einem Substanzgemisch verwendet wird.

14. Expressionsvektor für die Expression eines Fusionsgens unter der Kontrolle eines exogenen induzierbaren Promotors, in welchem in operativer Verknüpfung mit dem Promotor eine kodierende Sequenz eines gewünschten Passagierpeptids im durchgehenden Leseraster stromabwärts zu einer kodierenden Sequenz für ein im carboxylterminalen Bereich von 280 Aminosäuren um wenigstens die D3-Domäne verkürztes Intimin als Verankerungsdomäne für das Passagierpeptid vorliegt.

15. Expressionsvektor nach Anspruch 14, wobei der Promotor ausgewählt ist aus der Gruppe lac-Promotor, ara-Promotor, tetA-Promotor.

16. Gramnegatives Wirtsbakterium der Gattung Enterobactericeae, transformiert mit wenigstens einem Expressionsvektor nach Anspruch 14.

## Claims

1. Method for presentation of peptides and proteins on the surface of host bacteria, wherein (a) a Gram-negative host bacterium is provided, which is transformed with a vector on which is located, in operative linkage with an exogenously inducible promoter, a fused nucleic acid sequence which: (i) contains a sequence section which codes an intimin shortened by at least the D3 domain in the carboxy terminal region, as an anchoring domain, and (ii) a nucleic acid sequence section which codes for the passenger peptide or passenger polypeptide/protein to be presented, and (b) the host bacterium is cultured under conditions under which expression of the fused nucleic acid sequence and presentation of the peptide/polypeptide/protein coded by the nucleic acid sequence section (ii) on the surface of the host bacterium take place, the nucleic acid sequence section (ii) being heterologous with respect to the nucleic acid sequence section which codes for the intimin membrane anchoring domain.

2. Method according to claim 1, **characterized in that** the shortened intimin is shortened in the carboxy terminal region of 280 amino acids by at least one further of the intimin domains D0, D1 or D2.

3. Method according to claim 1, **characterized in that** the intimin anchoring domain used has been derived in the outer bacteria membrane from the genus of Enterobacteriaceae and is used in a host bacterium of a genus of Enterobacteriaceae.

4. Method according to claim 3, **characterized in that** a fragment of the intimin from enterohaemorrhagic E. coli or a variant thereof is used as the anchoring domain.

5. Method according to claim 4, **characterized in that** a carboxy-terminally shortened variant of the EaeA intimin from enterohaemorrhagic Escherichia coli 0157: H7 is used as the anchoring domain.

6. Method according to claim 5, **characterized in that** the shortened variant of the EaeA intimin contains amino acids 1 to 659.

7. Method according to claim 5, **characterized in that** the shortened variant of the EaeA intimin contains amino acids 1 to 753.

8. Method according to claim 1, in which the expression of the fusion gene from the nucleic acid sequence section which codes for the shortened intimin and that which codes for the passenger protein can be regulated, wherein (i) a glutamine-coding codon of the nucleic acid sequence section which codes for the shortened intimin is replaced by an amber stop codon (TAG) and (ii) an Escherichia coli host strain is used in which translation of the mRNA of the fusion gene takes place by provision of a regulatable amount of suppressor tRNA which renders possible over-reading of the stop codon during the translation.

9. Method according to claim 8, in which the regulation of the expression of the gene for the amber suppressor tRNA takes place by this being placed under the control of a promoter which can be regulated in its transcription rate.

10. Method according to claim 9, in which the promoter which can be regulated in its transcription rate is the PLlac promoter.

11. Method for the preparation of a variant population of surface-exposed peptides/polypeptides/proteins and for the identification of bacteria which carry the peptides/polypeptides/proteins with a particular desired property, wherein the method comprises the following steps: (i) preparation of one or more fusion genes or fusion gene fragments by cloning of the coding sequence of a desired passenger in the uninterrupted reading frame with the coding sequence of the intimin gene shortened by at least the D3 domain in the carboxy terminal region into at least one expression vector; (ii) variation of the passenger peptide by one of the methods chosen from: targeted site-directed mutagenesis, e.g. by a polymerase chain reaction (PCR) using oligonucleotides with bases exchanged in a targeted manner, by random mutagenesis using oligonucleotide mixtures with randomly generated base sequences in selected sequence sections in the PCR, by error-prone PCR, randomly controlled chemical or radiation-generated mutagenesis, (iii) introduction of the at least one vector into host bacteria which present the passenger stably on their surface, (iv) expression of the fusion gene in the host bacteria, (v) culture of the bacteria for production of a passenger presented stably exposed on the surface, and (vi) selective concentration of the bacteria which carry the passenger having the desired properties on the surface.

12. Method according to claim 11, in which the identification of the bacterium which present, exposed on the surface, a passenger having a desired binding affinity takes place by binding to an immobilized and/or labelled binding partner.

13. Method according to claim 11, in which a population of bacteria which carry, exposed on the surface, a passenger having a binding affinity for a binding partner is used as the matrix for purification of the binding partner from a substance mixture by affinity chromatography.

14. Expression vector for the expression of a fusion gene under the control of an exogenous inducible promoter, in which, in operative linkage with the promoter, a coding sequence of a desired passenger peptide is present in the uninterrupted reading frame downstream of a coding sequence for an intimin shortened by at least the D3 domain in the carboxy terminal region of 280 amino acids, as the anchoring domain for the passenger peptide.

15. Expression vector according to claim 14, wherein the promoter is chosen from the group of lac promoter, ara promoter and tetA promoter.

16. Gram-negative host bacterium of the genus Enterobacteriaceae, transformed with at least one expression vector according to claim 14.

## Revendications

1. Procédé pour la présentation de peptides et de protéines à la surface de bactéries hôtes, dans lequel (a) on fournit une bactérie hôte à Gram négatif qui est transformée par un vecteur sur lequel est localisée une séquence d'acide nucléique fusionnée, en liaison opérationnelle avec un promoteur inductible exogène, la séquence d'acide nucléique fusionnée comprenant : (i) un segment de séquence qui code pour une intimine raccourcie d'au moins le domaine D3 dans la région carboxy-terminale, en tant que domaine d'ancrage et (ii) un segment de séquence d'acide nucléique codant pour le peptide passager ou le polypeptide passager/la protéine passagère à présenter, et (b) on cultive la bactérie hôte dans des conditions dans lesquelles une expression de la séquence d'acide nucléique fusionnée et une présentation du peptide/polypeptide/de la protéine codés par le segment de séquence d'acide nucléique (ii) s'effectuent à la surface de la bactérie hôte, le segment de séquence d'acide nucléique (ii) étant hétérologue par rapport au segment de séquence d'acide nucléique codant pour le domaine d'ancrage à la membrane de l'intimine.

2. Procédé selon la revendication 1, **caractérisé en ce que** l'intimine raccourcie est raccourcie d'au moins un autre des domaines de l'intimine D0, D1, D2 dans la région carboxy-terminale de 280 acides aminés.

3. Procédé selon la revendication 1, **caractérisé en ce que** le domaine d'ancrage de l'intimine dans la membrane bactérienne externe utilisé a été issu de la famille des *Enterobacteriaceae* et est utilisé dans une bactérie hôte appartenant à la famille des *Enterobacteriaceae.*

4. Procédé selon la revendication 3, **caractérisé en ce qu'**on utilise comme domaine d'ancrage un fragment de l'intimine provenant de *E. coli* entérohémorragique ou un variant de celui-ci.

5. Procédé selon la revendication 4, **caractérisé en ce qu'**on utilise comme domaine d'ancrage une variante raccourcie à l'extrémité carboxy-terminale de l'intimine EaeA provenant d'*Escherichia coli* entérohémorragique O157:H7.

6. Procédé selon la revendication 5, **caractérisé en ce que** la variante raccourcie de l'intimine EaeA comporte les acides aminés 1 à 659.

7. Procédé selon la revendication 5, **caractérisé en ce que** la variante raccourcie de l'intimine EaeA comporte les acides aminés 1 à 753.

8. Procédé selon la revendication 1, dans lequel l'expression du gène de fusion constitué du segment de séquence d'acide nucléique codant pour l'intimine raccourcie et du segment de séquence d'acide nucléique codant pour la protéine passagère est régulable, dans lequel (i) un codon codant pour une glutamine du segment de séquence d'acide nucléique codant pour l'intimine raccourcie est remplacé par un codon stop ambre (TAG) et (ii) on utilise une souche hôte de *E. coli,* dans laquelle une traduction de l'ARNm du gène de fusion a lieu par apport d'une quantité régulable d'ARNt suppresseur, qui permet un saut de lecture du codon stop lors de la traduction.

9. Procédé selon la revendication 8, dans lequel la régulation de l'expression du gène pour l'ARNt suppresseur ambre a lieu par le fait que celui-ci est mis sous contrôle d'un promoteur régulable dans sa vitesse de transcription.

10. Procédé selon la revendication 9, dans lequel le promoteur régulable dans sa vitesse de transcription est le promoteur PL1ac.

11. Procédé pour la production d'une population variante de peptides/polypeptides/protéines exposés à la surface et pour l'identification de bactéries qui portent des peptides/polypeptides/protéines ayant respectivement une propriété désirée, le procédé comprenant les étapes suivantes: (i) production d'un ou plusieurs gènes de fusion ou fragments de fusion par clonage de la séquence codante d'un passager désiré en phase avec le cadre de lecture de la séquence codante du gène de l'intimine raccourcie d'au moins le domaine D3 dans la région carboxy-terminale, dans au moins un vecteur d'expression ; (ii) variation du peptide passager par une méthode choisie parmi : la mutagenèse ciblée, dirigée, par exemple par réaction d'amplification en chaîne par polymérase (PCR) avec utilisation d'oligonucléotides comportant des bases choisies de façon ciblée, par mutagenèse aléatoire avec utilisation de mélanges d'oligonucléotides avec des successions de bases produites au hasard dans des segments de séquences choisis dans la PCR, par PCR *error-prone* (sujette à erreurs), mutagenèse aléatoire engendrée par voie chimique ou par irradiation, (iii) introduction d'au moins un vecteur dans des bactéries hôtes qui présentent de façon stable le passager à leur surface, (iv) expression du gène de fusion dans les bactéries hôtes, (v) culture des bactéries pour la production d'un passager présenté, exposé de façon stable à la surface, et (vi) enrichissement sélectif en les bactéries qui portent à la surface le passager ayant les propriétés désirées.

12. Procédé selon la revendication 11, dans lequel l'identification de la bactérie, qui présente un passager exposé à la surface avec une affinité de liaison désirée, se fait par liaison à un partenaire de liaison immobilisé et/ou marqué.

13. Procédé selon la revendication 11, dans lequel on utilise une population de bactéries, qui portent exposé à la surface un passager ayant une affinité de liaison pour un partenaire de liaison, comme matrice pour la purification par chromatographie d'affinité du partenaire de liaison à partir d'un mélange de substances.

14. Vecteur d'expression pour l'expression d'un gène de fusion sous le contrôle d'un promoteur inductible exogène, dans lequel une séquence codante d'un peptide passager, en phase avec le cadre de lecture en aval d'une séquence codant pour une intimine raccourcie d'au moins le domaine D3 dans la région carboxy-terminale de 280 acides aminés comme domaine d'ancrage pour le peptide passager, est présente en liaison opérationnelle avec le promoteur.

15. Vecteur d'expression selon la revendication 14, dans lequel le promoteur est choisi dans le groupe constitué du promoteur lac, du promoteur ara, du promoteur tetA.

16. Bactérie hôte à Gram négatif, appartenant à la famille des *Enterobacteriaceae,* transformée par au moins un vecteur d'expression selon la revendication 14.
